# EUROPEAN PATENT APPLICATION

(11) **EP 3 536 352 A1**
(43) Date of publication of application: **11.09.2019**
(21) Application number: 17867825.6
(22) Date of filing: 07.11.2017
(51) Int. Cl.: A61L 27/20, A61K 9/00, A61L 27/04, A61L 27/36, A61L 27/50, A61L 27/54

(54) **KERATOCONJUNCTIVAL COVER SHEET AND METHOD FOR PRODUCING KERATOCONJUNCTIVAL COVER SHEET**

(30) Priority: 07.11.2016 JP 2016217415
(71) Applicant: Osaka University, Suita-shi, Osaka 565-0871 (JP)
(72) Inventor: BABA, Koichi, Suita-shi Osaka 565-0871 (JP); NISHIDA, Kohji, Suita-shi Osaka 5650871 (JP)
(74) Representative: Prinz & Partner mbB
(86) International application number: PCT/JP2017/040057
(87) International publication number: WO 2018/084306

(57) **Abstract**

Provided is an inexpensive keratoconjunctival cover sheet that is comfortable and has good oxygen-permeability, contamination-resistance, wettability, handleability, and optical properties, and that can be used for medical treatment, visual correction, a cosmetic application, and the like. The keratoconjunctival cover sheet is constituted by a gel layer that includes metal ions and a water-soluble gelling agent exhibiting a gelation ability in the presence of the metal ions, and has a surface area capable of covering a cornea or keratoconjunctiva.

## Description

### Technical Field

The present disclosure relates to a keratoconjunctival cover sheet and a method for producing a keratoconjunctival cover sheet.

### Background Art

Ophthalmic pharmaceuticals and medical instruments to be used for treatment, prevention, and diagnosis of diseases, visual correction, a cosmetic application, and the like have undergone development. Examples thereof include eye-drop medicines, ophthalmic ointments, and contact lenses. Eye-drop medicines and ophthalmic ointments are medicines to be applied to an eye for the purpose of treatment of eye diseases, and their effective components move to the anterior eye chamber, the ciliary body, the crystalline lens, and the like inside the eye mainly through the cornea or the keratoconjunctiva (cornea and conjunctiva). Contact lenses are medical instruments to be brought into contact with and worn on the corneas or keratoconjunctivas, and are used in a wide variety of applications. Examples of the applications include visual correction, cornea protection, disease treatment applications typified by contact lenses for sustained drug release, and cosmetic applications typified by color contact lenses. The cornea constitutes a front surface of the outer layer of the ocular bulb wall and is exposed from the body surface, and therefore, the shape of the cornea or keratoconjunctiva, contact with the cornea or keratoconjunctiva, or the like needs to be taken into consideration when ophthalmic pharmaceuticals and medical instruments are designed.

Ophthalmic pharmaceuticals in the form of an eye-drop medicine are often used because eye-drop medicines are easy to handle. An administered eye-drop medicine mixes with a lacrimal fluid, and most of the mixture accumulates in the conjunctival sac of the lower eyelid. The pharmaceutical component moves into the eye and exhibits its medicinal effect. However, after ocular instillation, the eye-drop medicine is discharged together with the lacrimal fluid to the lacrimal sac through the lacrimal puncta at the upper and lower inner eyelid edges via the lacrimal canaliculi due to several blinks, and therefore, the bioavailability of the eye-drop medicine is very low. On the other hand, the frequent application of a large amount of an eye-drop medicine to the eye may cause a systemic side-effect caused by absorption of the pharmaceutical component through the mucosa of the lacrimal sac. Accordingly, studies for improving the persistence of the medicinal effects of ophthalmic pharmaceuticals and increasing the bioavailability of the drug are ongoing.

For example, a long-acting ophthalmic preparation utilizing gelation of an aqueous solution of alginic acids such as sodium alginate caused by calcium ions contained in a biogenic fluid such as a lacrimal fluid has been reported (Patent Documents 1 and 2, and Non-Patent Document 1). It is known that when alginic acids come into contact with polyvalent positive ions in a solution, the solution gelates through ion-crosslinking of a plurality of alginic acid molecules. Regarding the techniques disclosed in Patent Documents 1 and 2 and Non-Patent Document 1 in which such alginic acids are applied ophthalmologically, alginic acids including guluronic acid at a ratio greater than or equal to a certain ratio are used. When alginic acids come into contact with a biogenic fluid, the solution gelates and stays in an application site, and therefore, the active component such as a drug contained in the ophthalmic preparation will act continuously, thus making it possible to improve the bioavailability of the drug.

The lacrimal fluid is secreted by the lacrimal gland, moistens the surfaces of the cornea and conjunctiva due to blinking, and accumulates in the conjunctival sac, while the lacrimal fluid is discharged to the lacrimal sac through the lacrimal puncta at the upper and lower inner eyelid edges via the lacrimal canaliculi. As described above, an ophthalmic preparation mixes with the lacrimal fluid when applied to the eye, and most of the mixture accumulates in the conjunctival sac of the lower eyelid. Therefore, regarding the technologies disclosed in Patent Documents 1 and 2 and Non-Patent Document 1, it is anticipated that the drug treatment effect will be limited because most of the alginic acids are involved in gelation in the conjunctival sac of the lower eyelid and the resulting gel stays there.

Regarding contact lenses serving as ophthalmic medical instruments, research and development based on known manufacturing techniques and materials are ongoing in order to improve the functions of contact lenses. For example, a lathe cutting method, a spin casting method, and a molding method are mainly used as a method for manufacturing contact lenses. With the lathe cutting method, contact lenses are manufactured by processing a large contact lens material into a rod shape and cutting and grinding it. With the spin casting method, an unpolymerized liquid contact lens material is poured into a rotational concave mold and polymerized while rotated, and then the material is spread using centrifugal force and shaped into a lens shape. With the molding method, an unpolymerized liquid contact lens material is poured into a concave mold, and after a convex mold is then fit thereto, the material is polymerized and shaped into a lens shape. Examples of conventional materials of hard contact lenses include non-oxygen-permeable materials such as methyl methacrylate and oxygen-permeable materials such as siloxanyl methacrylate, and examples of conventional materials of soft contact lenses include hydrous materials such as hydroxyethyl methacrylate, anhydrous materials such as silicone rubber, and silicone hydrogel obtained by combining silicone having very high oxygen-permeability with hydrogel.

However, many people who use hard contact lenses made of a hard material complain of a feeling that a foreign material is present. On the other hand, soft contact lenses are softer than hard contact lenses and thus are relatively comfortable, but there is demand for further improvement in the comfort. It is desirable to secure a sufficient amount of oxygen to be supplied to the corneas and keratoconjunctivas during use of contact lenses, but the oxygen-permeability of water-containing soft contact lenses depends on the water content ratio, and thus there is demand for an increase in the water content ratio. On the other hand, oxygen-permeable hard contact lenses, and soft contact lenses made of a silicone hydrogel material have high oxygen-permeability, but many materials having high oxygen-permeability are lipophilic, and a silicone-based material, which is a constituent component of the silicone hydrogel material, is also lipophilic. There is a problem in that, due to such a lipophilic material on the surfaces of contact lenses, the lenses absorb biological components such as lipids and proteins derived from the lacrimal fluid and eye discharge and cosmetic components typified by an eye makeup such as an eye shadow or a mascara, and are thus contaminated. It is reported that, in order to continuously hold the contamination-resistance and the wettability of the contact lenses, an ionic hydrophilic macromolecule such as alginic acid is included in deoxidation and desalting treatment for counter-ions from a cationic monomer such as dimethylaminoethyl methacrylate methyl hydroxide and an anionic monomer such as methacrylic acid, which are used as constituent base materials (see Patent Document 3). However, conventional materials are used as the base materials, and therefore, the above-mentioned problem is still included. In addition, it is known that use of contact lenses causes a dry-eye condition.

Contact lenses are applied on the corneas, which poses problems in that applying and removing contact lenses takes some getting used to and takes effort, and elderly persons and the like are likely to avoid use of contact lenses.

There is also a problem in that when contact lenses are constructed for visual correction, and, for example, visual correction for both near vision and far vision is required, users are not sufficiently satisfied due to visual correction for one of near vision and far vision being insufficient, for example. Accordingly, there is room for further improvement in materials and optical properties of conventional contact lenses.

### Citation List

### Patent Literature

Patent Document 1: JP 2002-332248A
Patent Document 2: WO 2008/001872
Patent Document 3: JP 2010-281956A

### Non-Patent Literature

Non-Patent Document 1: S. Cohen et al., "A novel in situ-forming ophthalmic drug delivery system from alginates undergoing gelation in the eye", HYPERLINK "http://www.sciencedirect.com/science/journal/01683659" \o "Go to Journal of Controlled Release on ScienceDirect" Journal of Controlled Release, 44, 1997, P201?208

### Summary of Invention

### Technical Problem

The present disclosure was achieved to solve the problems of conventional ophthalmic pharmaceuticals and ophthalmic medical instruments and provide an inexpensive keratoconjunctival cover sheet that is comfortable and has good oxygen-permeability, contamination-resistance, wettability, handleability, and optical properties, and that can be used for medical treatment, visual correction, or a cosmetic application. Furthermore, the present disclosure was also achieved to provide a method for producing a keratoconjunctival cover sheet with the above-mentioned properties.

### Solution to Problem

The inventors of the present invention conducted intensive studies in order to solve the above-mentioned problems, and found that a keratoconjunctival cover sheet constituted by a gel layer having a surface area capable of covering the cornea or keratoconjunctiva could be formed by utilizing the properties of ion-responsive gelling agents such as alginic acids, and this keratoconjunctival cover sheet could be formed through ocular instillation, which is a simple technique. Furthermore, the inventors of the present invention found that the keratoconjunctival cover sheet was comfortable, had good oxygen-permeability, contamination-resistance, wettability, handleability, and optical properties, and could be favorably used for medical treatment, visual correction, a cosmetic application, or the like, and achieved a keratoconjunctival cover sheet and a method for producing the keratoconjunctival cover sheet of the present disclosure based on these findings.

Specifically, in order to solve the above-mentioned problems, the invention including the aspects described in [1] to [15] below is provided.
[1] A keratoconjunctival cover sheet constituted by a gel layer that includes metal ions and a water-soluble gelling agent exhibiting a gelation ability in the presence of the metal ions, and has a surface area capable of covering a cornea or keratoconjunctiva.
[2] In the keratoconjunctival cover sheet, the water-soluble gelling agent is alginic acid or an alginic acid derivative, and the metal ions are positive ions or negative ions.
[3] In the keratoconjunctival cover sheet, the metal ions are calcium ions.
[4] The keratoconjunctival cover sheet is used for protection from an external environment.
[5] The keratoconjunctival cover sheet contains a therapeutically effective amount of a drug in the gel layer and is used to slowly release the drug.
[6] In the keratoconjunctival cover sheet, the gel layer is constituted by a contact lens with a predetermined refractive index.
[7] The keratoconjunctival cover sheet contains a water-soluble component with a high refractive index in the gel layer and is used for visual correction.
[8] In the keratoconjunctival cover sheet, the water-soluble component with a high refractive index is selected from sugars, polyhydric alcohols, water-soluble polymers, and mixtures thereof.
[9] In the keratoconjunctival cover sheet, the gel layer contains an oil component or a moisturizing component.
[10] A method for producing a keratoconjunctival cover sheet, including: a step of applying a water-soluble gelling agent to an eye, in which a water-soluble gelling agent exhibiting a gelation ability in the presence of metal ions is applied to an anterior ocular segment; and a step of applying metal ions to an eye, in which the metal ions are applied to the anterior ocular segment, wherein gelation by the water-soluble gelling agent is caused by the metal ions, and a gel layer is formed on a cornea or keratoconjunctiva.
[11] In the method for producing a keratoconjunctival cover sheet, the step of applying a water-soluble gelling agent to an eye includes a step of applying a therapeutically effective amount of a drug to the anterior ocular segment.
[12] In the method for producing a keratoconjunctival cover sheet, the step of applying a water-soluble gelling agent to an eye includes a step of applying a water-soluble component with a high refractive index to the anterior ocular segment.
[13] The method for producing a keratoconjunctival cover sheet includes a step of controlling a refractive index, in which gel layers including a single component or gel layers including a plurality of components are laminated by repeating the step of applying a water-soluble gelling agent to an eye and the step of applying metal ions to an eye to control a refractive index.
[14] The method for producing a keratoconjunctival cover sheet includes a step of controlling a refractive index, in which at least one of factors of the gel layer associated with a refractive index, such as viscosity, magnetic permeability, dielectric constant, electric conductivity, and gel thickness, is adjusted to control a refractive index.
[15] The method for producing a keratoconjunctival cover sheet further includes a step of applying an oil component or a moisturizing component to the anterior ocular segment.

With the configuration described in [1] above, a keratoconjunctival cover sheet constituted by a gel layer that is made of metal ions and a water-soluble gelling agent exhibiting a gelation ability in the presence of the metal ions and that has a surface area capable of covering a cornea or keratoconjunctiva can be provided. The keratoconjunctival cover sheet of the present disclosure is constituted by a gel layer that is mostly constituted by an aqueous solvent, and thus exhibits good oxygen-permeability and good wettability and can suppress ophthalmopathy such as dry-eye. The keratoconjunctival cover sheet of the present disclosure is highly hydrophilic and can thus suppress attachment of biological components such as lipids and proteins derived from lacrimal fluid and eye discharge, which are oleaginous components, and cosmetic components typified by an eye makeup such as an eye shadow or a mascara and may exhibit good contamination-resistance. Furthermore, the keratoconjunctival cover sheet of the present disclosure can be applied through ocular instillation, which is a simple technique, and the gel layer can be configured to naturally biodegrade in the eye and be discharged to the outside of the body. Therefore, the keratoconjunctival cover sheet of the present disclosure can be easily handled without troublesome work unlike a case where conventional contact lenses and the like are applied and removed. In addition, the gel layer is a soft material, and thus comfort can be achieved. Moreover, the keratoconjunctival cover sheet of the present disclosure is made of a water-soluble gelling agent and metal ions, and is thus very inexpensive. The keratoconjunctival cover sheet of the present disclosure with such properties can be favorably used for medical treatment, visual correction, a cosmetic application, or the like. Furthermore, the keratoconjunctival cover sheet of the present disclosure is formed through gelation by the water-soluble gelling agent caused by the addition of a sufficient amount of the metal ions, which is larger than the amount of metal ions contained in the lacrimal fluid. Accordingly, for example, compared with the above-mentioned cases disclosed in Patent Document 1, Patent Document 2, Non-Patent Document 1, and the like in which the gelation by a water-soluble gelling agent is caused by the lacrimal fluid, effects of increasing the gel strength, increasing the gel thickness, securing a cover area with respect to the cornea or keratoconjunctiva, and the like can be expected. Moreover, the keratoconjunctival cover sheet is constituted by the gel layer formed through the addition of the metal ions subsequent to the addition of the water-soluble gelling agent on the cornea or keratoconjunctiva, and therefore, it can be expected that a portion of the gel on a side on which the metal ions have been added, which comes into contact with air, will be firm and hard and suppress moisture evaporation, and a portion of the gel that comes into contact with the cornea or keratoconjunctiva will be soft and have improved comfort. In addition, the keratoconjunctival cover sheet of the present disclosure is constituted by the gel layer that gelates soon after the water-soluble gelling agent is dripped, and can thus favorably cover the cornea or keratoconjunctiva, unlike the above-mentioned cases disclosed in Patent Document 1, Patent Document 2, and Non-Patent Document 1 in which a water-soluble gelling agent is involved in gelation in the conjunctival sac of the lower eyelid where the lacrimal fluid accumulates, and the resulting gel stays there.

With the configuration described in [2] above, the keratoconjunctival cover sheet in which alginic acid or an alginic acid derivative is used as the water-soluble gelling agent can be provided. With the keratoconjunctival cover sheet of the present disclosure, high hydrophilicity of the alginic acid or alginic acid derivative makes it possible to achieve better comfort, oxygen-permeability, contamination-resistance, wettability, and handleability. Moreover, the alginic acid or alginic acid derivative is a naturally occurring substance, and thus has high biocompatibility and biodegradability and can be safely applied to the cornea or keratoconjunctiva. Furthermore, the alginic acid or alginic acid derivative is very inexpensive, and thus the cost effectiveness can be further improved.

With the configuration described in [3] above, the keratoconjunctival cover sheet in which calcium ions are used as the metal ions can be provided. The presence of calcium ions enables a gel to be instantly formed by alginic acid or an alginic acid derivative, and thus the keratoconjunctival cover sheet of the present disclosure can be stably provided. Moreover, calcium is an essential component for maintaining a living organism and is highly biocompatible, and therefore, the keratoconjunctival cover sheet of the present disclosure can be safely applied to the cornea or keratoconjunctiva. Furthermore, calcium is a metal that is abundantly present in nature and very inexpensive, and thus the cost effectiveness can be further improved.

With the configuration described in [4] above, a keratoconjunctival cover sheet for protecting the cornea or keratoconjunctiva from the external environment can be provided. The keratoconjunctival cover sheet can be used to prevent the occurrence of allergic diseases such as allergic conjunctivitis, to prevent postoperative infection of a cornea or keratoconjunctiva, and the like.

With the configuration described in [5] above, a therapeutically effective amount of a drug can be added to the gel layer, and thus a keratoconjunctival cover sheet that can be favorably used for treatment, prevention, and diagnosis of ocular diseases can be provided. The keratoconjunctival cover sheet of the present disclosure covers the surface of the cornea or keratoconjunctiva along the shape of the cornea or keratoconjunctiva, and therefore, the drug can be directly and efficiently applied to the cornea or keratoconjunctiva, and thus the therapeutic effect can be improved. Furthermore, the drug release speed can be controlled due to the gel layer, thus making it possible to improve the bioavailability of the drug and reduce side-effects. Moreover, the medication compliance can also be improved.

With the configuration described in [6] above, a keratoconjunctival cover sheet configured as a contact lens with a refractive index can be provided. The keratoconjunctival cover sheet of the present disclosure can be used as a contact lens that is comfortable and has good oxygen-permeability, contamination-resistance, wettability, handleability, and optical properties. The keratoconjunctival cover sheet can be used as a soft contact lens for myopia correction, hyperopia correction, myopia-hyperopia correction, and the like.

With the configuration described in [7] above, a water-soluble component with a high refractive index can be added to the gel layer, and a keratoconjunctival cover sheet that can be favorably used for visual correction can be provided. The gel layer constituting the keratoconjunctival cover sheet of the present disclosure is mostly constituted by water, and therefore, the refractive index of the gel layer can be controlled according to the type, concentration, and the like of the water-soluble component with a high refractive index. Accordingly, tailor-made contact lenses that can be favorably used as soft contact lenses for myopia correction, hyperopia correction, myopia-hyperopia correction, and the like and that are optimized for users can be provided.

With the configuration described in [8] above, a keratoconjunctival cover sheet in which a sugar, a polyhydric alcohol, a water-soluble polymer, or a mixture thereof is used as the water-soluble component with a high refractive index and that can be favorably used for visual correction can be provided. Sugars, polyhydric alcohol, and water-soluble polymers have a high refractive index and are favorably mixed or dissolved in water, and therefore, the refractive index of the keratoconjunctival cover sheet of the present disclosure can be effectively controlled. These substances are biocompatible and biodegradable enough to be used as food additives, and can be safely applied to the cornea or keratoconjunctiva. Furthermore, these substances are very inexpensive, and thus the cost effectiveness can be further improved.

With the configuration described in [9] above, a keratoconjunctival cover sheet can be provided in which moisture evaporation from the inside of the gel layer is suppressed and water retention is improved while the functions are favorably maintained, thus making it possible to effectively suppress the induction of a dry-eye condition and the like caused by acceleration of the evaporation of the lacrimal fluid and dryness of the eye, which have been known to be induced by wearing conventional contact lenses.

With the configuration described in [10] above, a method for producing a keratoconjunctival cover sheet including a gel layer made of a water-soluble gelling agent exhibiting a gelation ability in the presence of metal ions and the metal ions. The method for producing a keratoconjunctival cover sheet of the present disclosure uses ocular instillation, which is a simple technique, and does not require technical skills and the like. The method also does not require a large-scale production apparatus or the like, and thus a keratoconjunctival cover sheet can be provided at very low cost. With the method for producing a keratoconjunctival cover sheet of the present disclosure, the application by a subject is simultaneous with the production, and the application site is used as a mold, thus making it possible to provide a tailor-made keratoconjunctival cover sheet suited for the ocular structure (particularly the shapes of the cornea and the palpebral conjunctiva) of the user. Furthermore, with the method for producing a keratoconjunctival cover sheet of the present disclosure, gelation by the water-soluble gelling agent is caused by the addition of a sufficient amount of the metal ions, which is larger than the amount of metal ions contained in the lacrimal fluid. Accordingly, for example, compared with the above-mentioned cases disclosed in Patent Document 1, Patent Document 2, Non-Patent Document 1, and the like in which the gelation by a water-soluble gelling agent is caused by the lacrimal fluid, effects of increasing the gel strength, increasing the gel thickness, securing a cover area with respect to the cornea or keratoconjunctiva, and the like can be expected. Moreover, with the method for producing a keratoconjunctival cover sheet of the present disclosure, the gel layer is formed through the addition of the metal ions subsequent to the addition of the water-soluble gelling agent on the cornea or keratoconjunctiva, and it is expected that a portion of the gel on a side on which the metal ions have been added, which comes into contact with air, will be firm and hard and suppress moisture evaporation, and a portion of the gel that comes into contact with the cornea or keratoconjunctiva will be soft and have improved comfort. In addition, the gel can be formed soon after the water-soluble gelling agent is dripped, and can thus favorably cover the cornea or keratoconjunctiva, unlike the above-mentioned cases disclosed in Patent Document 1, Patent Document 2, and Non-Patent Document 1 in which a water-soluble gelling agent is involved in gelation in the conjunctival sac of the lower eyelid where the lacrimal fluid accumulates, and the resulting gel stays there.

With the configuration described in [11] above, a method for producing a keratoconjunctival cover sheet in which a therapeutically effective amount of a drug is added to the gel layer and that can be favorably used for treatment, prevention, and diagnosis of ocular diseases can be provided. The keratoconjunctival cover sheet produced using the method for producing a keratoconjunctival cover sheet of the present disclosure covers the surface of the cornea or keratoconjunctiva along the shape of the cornea or keratoconjunctiva, and therefore, the drug can be directly and efficiently applied to the cornea or keratoconjunctiva, and thus the therapeutic effect can be improved. Furthermore, the drug release speed can be controlled due to the gel layer, thus making it possible to improve the bioavailability of the drug and reduce side-effects. Moreover, the medication compliance can also be improved. Moreover, the medication compliance can also be improved.

With the configuration described in [12] above, a method for producing a keratoconjunctival cover sheet in which a water-soluble component with a high refractive index can be added to the gel layer and that can be favorably used for visual correction can be provided. The gel layer constituting the keratoconjunctival cover sheet produced using the method for producing a keratoconjunctival cover sheet of the present disclosure is mostly constituted by water, and therefore, the refractive index of the gel layer can be controlled according to the type, concentration, and the like of the water-soluble component with a high refractive index. Accordingly, tailor-made contact lenses that can be favorably used as soft contact lenses for myopia correction, hyperopia correction, myopia-hyperopia correction, and the like and that are optimized for users can be provided.

With the configuration described in [13] above, a method for producing a keratoconjunctival cover sheet in which the gel layers including a single component or gel layers including a plurality of components are stacked and that can be favorably used for visual correction can be provided. With the keratoconjunctival cover sheet produced using the method for producing a keratoconjunctival cover sheet of the present disclosure, the light refractive index changes due to an interface between the gel layers, and the refractive index of the keratoconjunctival cover sheet can be appropriately controlled.

With the configuration described in [14] above, a method for producing a keratoconjunctival cover sheet in which the factors of the gel layer such as the viscosity, the magnetic permeability, the dielectric constant, the electric conductivity, and the gel thickness that are associated with the refractive index are adjusted and that can be favorably used for visual correction can be provided. The refractive index of the keratoconjunctival cover sheet produced using the method for producing a keratoconjunctival cover sheet of the present disclosure can be appropriately controlled by adjusting the above-mentioned factors associated with the refractive index as appropriate.

With the configuration described in [15] above, a method for producing a keratoconjunctival cover sheet in which moisture evaporation from the inside of the gel layer is suppressed and water retention is improved while the functions are favorably maintained can be provided, and a keratoconjunctival cover sheet can be provided that can effectively suppress the induction of a dry-eye condition and the like caused by acceleration of the evaporation of the lacrimal fluid and dryness of the eye, which have been known to be induced by wearing conventional contact lenses.

### Brief Description of Drawings

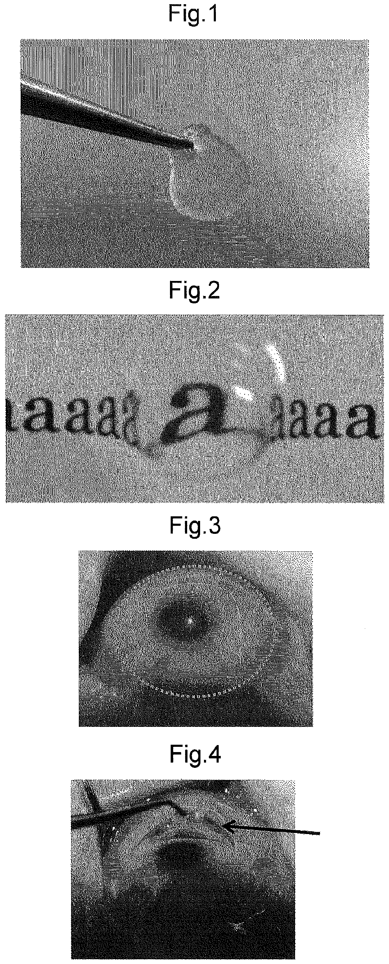

FIG. 1 is a diagram showing the result from in-vitro examination of production of a keratoconjunctival cover sheet in Example 1. The produced keratoconjunctival cover sheet was pinched using tweezers in order to confirm its gel strength.
FIG. 2 is a diagram showing the result from in-vitro examination of production of a keratoconjunctival cover sheet in Example 1. It was confirmed that the produced keratoconjunctival cover sheet had a lens-like function.
FIG. 3 is a diagram showing the result from in-vivo examination of production of a keratoconjunctival cover sheet in Example 2. It was confirmed that a keratoconjunctival cover sheet in the form of a circular gel could be formed on the keratoconjunctiva through ocular instillation.
FIG. 4 is a diagram showing the result from in-vivo examination of production of a keratoconjunctival cover sheet in Example 2. The keratoconjunctival cover sheet produced on the keratoconjunctiva was pinched using tweezers in order to confirm its gel strength.

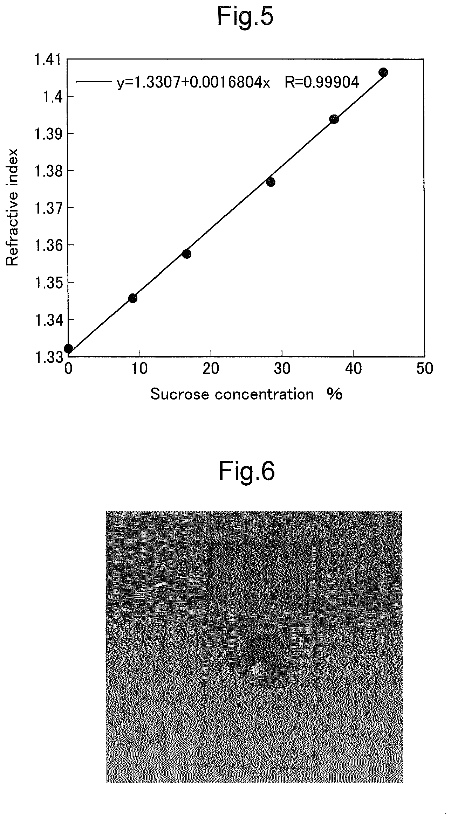

FIG. 5 is a graph showing the result from examination of the refractive index adjustment of a keratoconjunctival cover sheet in Example 3. It was confirmed that the refractive index of the keratoconjunctival cover sheet varied in the concentration of sucrose, which was a water-soluble component with a high refractive index, in a concentration-dependent manner.
FIG. 6 is a diagram showing the result from examination of coloring of a keratoconjunctival cover sheet in Example 4. It was confirmed that the keratoconjunctival cover sheet could be colored with a colorant such as a food dye.

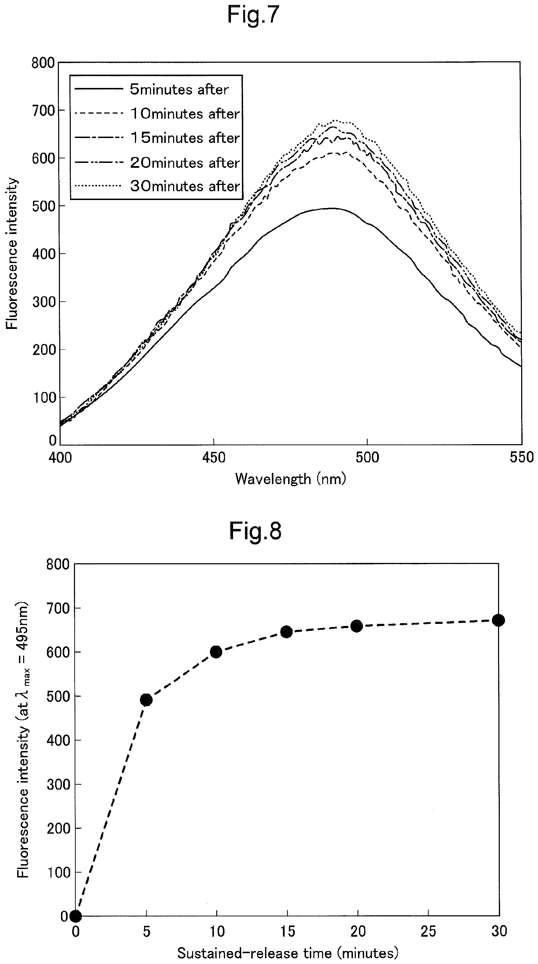

FIG. 7 is a graph (a temporal change in a fluorescence spectrum) showing the result from examination of a sustained-release effect of a keratoconjunctival cover sheet in Example 5. It was confirmed through fluorescence intensity measurement that a drug was slowly released from the keratoconjunctival cover sheet.
FIG. 8 is a graph (a temporal change in the maximum value) showing the result from examination of the refractive index adjustment of a keratoconjunctival cover sheet in Example 5, in which a temporal change in the maximum value in FIG. 7 is plotted. The behavior of a drug that was slowly released from the keratoconjunctival cover sheet was confirmed.

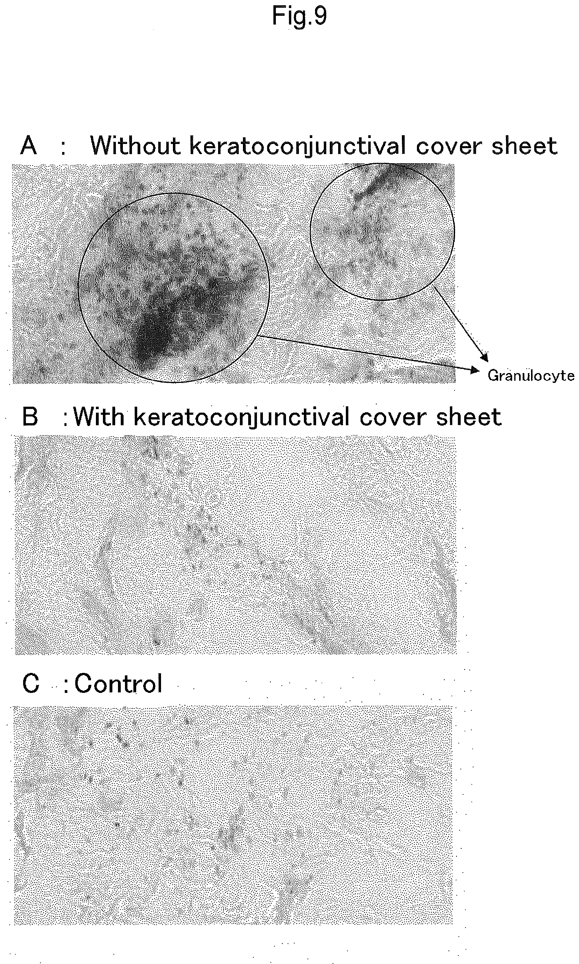

FIG. 9 shows micrographs of Giemsa-stained conjunctival portions, showing the results from in-vivo examination of an anti-allergic effect of a keratoconjunctival cover sheet in Example 6. Panel A shows the result from group (A) that was not provided with the keratoconjunctival cover sheet. Panel B shows the result from test group (B) that was provided with the keratoconjunctival cover sheet. Panel C shows the result from control group (C) that was not subjected to antigen challenge. Gray granules in the diagrams represent the presence of granulocytes of leukocyte.

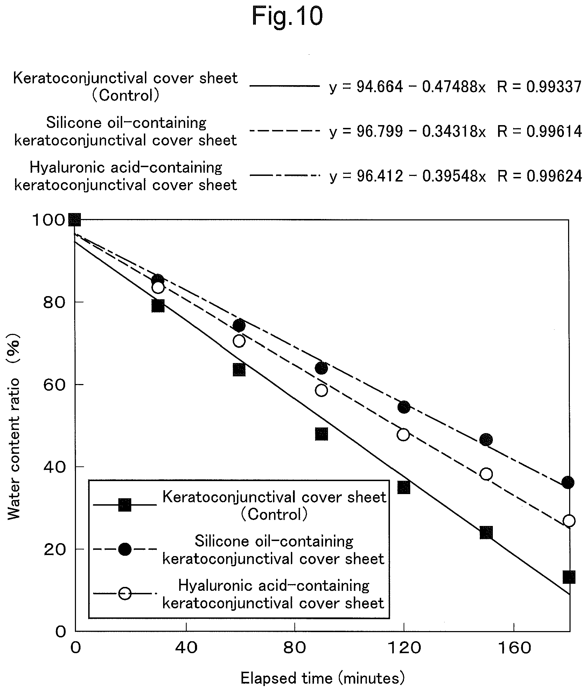

FIG. 10 is a graph showing the results from examination of a water-retention effect of a keratoconjunctival cover sheet in Example 7. It was confirmed that water evaporation from the keratoconjunctival cover sheet could be controlled by adding an additive to the keratoconjunctival cover sheet.

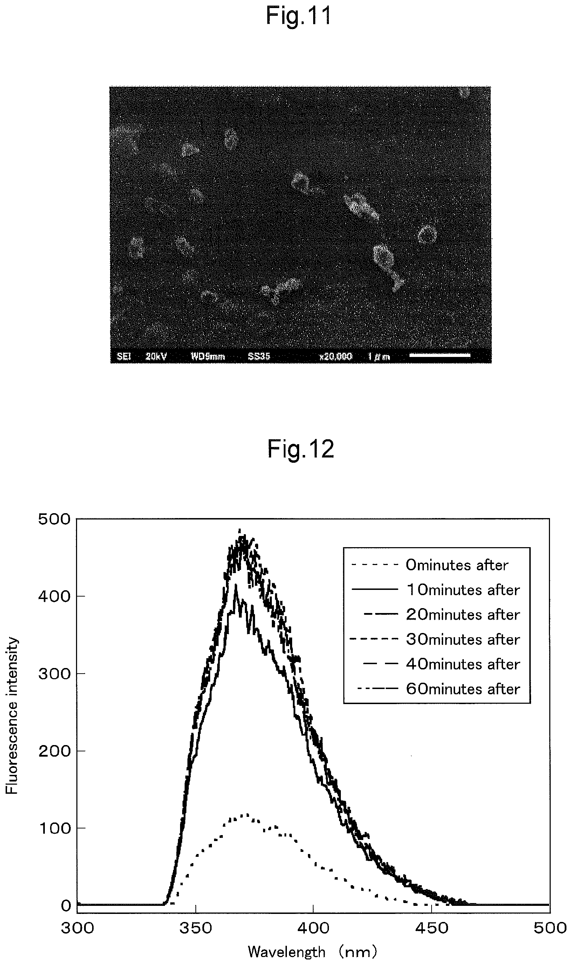

FIG. 11 is a scanning electron micrograph of nanoparticles of a therapeutic drug for dry eye, rebamipide. In Example 8, a sustained drug release effect of a keratoconjunctival cover sheet was examined using rebamipide as a drug for examination, and nanoparticles of rebamipide were confirmed under a scanning electron microscope.
FIG. 12 is a graph (a temporal change in a fluorescence spectrum) showing the result from examination of a sustained drug release effect of a keratoconjunctival cover sheet in Example 8. It was confirmed through fluorescence intensity measurement that rebamipide was slowly released from the keratoconjunctival cover sheet containing nanoparticles of rebamipide, which is used as a therapeutic drug for dry eye.

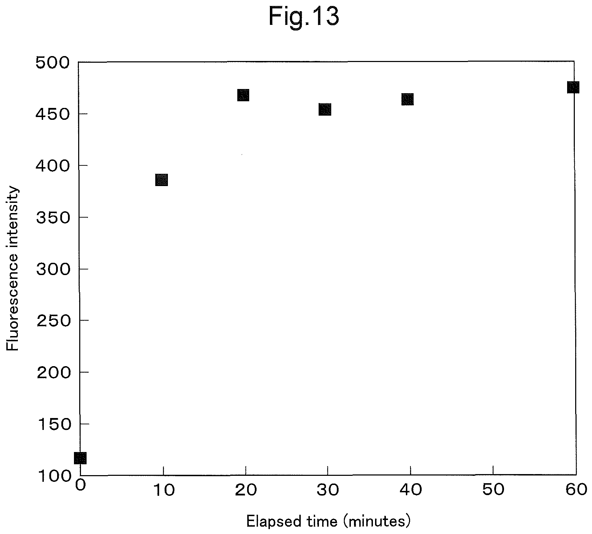

FIG. 13 is a graph (a temporal change in the maximum value) showing the result from examination of a sustained drug release effect of a keratoconjunctival cover sheet in Example 8, in which a temporal change in the maximum value in FIG. 12 is plotted. The behavior of rebamipide that was slowly released from the keratoconjunctival cover sheet was confirmed.

### Description of Embodiments

### Keratoconjunctival cover sheet

A keratoconjunctival cover sheet of this embodiment is constituted by a gel layer that includes metal ions and a water-soluble gelling agent exhibiting a gelation ability in the presence of the metal ions and that has a surface area capable of covering a cornea or keratoconjunctiva. Here, the term "keratoconjunctiva" refers to a cornea and a keratoconjunctiva.

Examples of the targets of the keratoconjunctival cover sheet of this embodiment include animals such as humans, non-human primates, rabbits, rats, guinea pigs, mice, dogs, cats, cats, horses, cows, pigs, sheep, goats, and chickens. Humans are particularly preferable.

The gel layer constituting the keratoconjunctival cover sheet of this embodiment is a physical gel in which the water-soluble gelling agent forms reversible crosslinks via the metal ions. The gel layer is a structure that has a skeleton with a three-dimensional network structure formed through the formation of partial intramolecular or intermolecular bonds of the gelling agent via the crosslinks between specific sites of the molecule, and that retains a large amount of an aqueous solvent that has lost its fluidity in the spaces formed in the skeleton. The gel layer encompasses a state in which only the surface gelates, and an aqueous solvent and an aqueous solution of the water-soluble gelling agent and the metal ions, which is obtained by dissolving the water-soluble gelling agent and the metal ions in the aqueous solvent, and the like are retained in regions surrounded by the gel, and a state in which the gel layer absorbs the aqueous solvent and swells, but does not dissolve. The term "gelation" means that the gelling agent forms the above-mentioned three-dimensional network structure that is insoluble or poorly soluble in the aqueous solvent through the formation of partial intramolecular or intermolecular bonds via the crosslinks between specific sites of the molecule. Water, purified water, a physiological saline solution, or the like can be used as the aqueous solvent.

The gel layer has a very high water content ratio of preferably 60 to 98%, and particularly preferably 70 to 95%, or 80 to 90%, and has excellent oxygen-permeability, wettability, and contamination-resistance to oleaginous components. It is preferable that the gel layer is very soft due to the high water content ratio and can thus be smoothly deformed by external force, and can be restored to the original state by the elastic force of the three-dimensional structure included in the gel layer when the external force is removed. Accordingly, comfort is achieved without interference with an eyelid opening-closing movement and eye movement. The gel layer may be configured to biodegrade with time in the eye, and can be configured to biodegrade in about 48 to 72 hours, for example. Furthermore, the gel layer may be configured such that its three-dimensional structure degrades when an appropriate chelating agent chelates the metal ions via which the crosslinks are formed by the water-soluble gelling agent. Examples of the chelating agent include EDTA, EGTA, and citric acid. It is preferable that the type and concentration of the water-soluble gelling agent, the type and concentration of the metal ions, and the like are changed as appropriate in accordance with the purpose or the like of the keratoconjunctival cover sheet, and external shape retention time and biodegradation time of the gel layer are thus determined as appropriate.

The shape of the gel layer has a surface area capable of covering a portion or all of the surface of the cornea or the keratoconjunctiva in the anterior ocular segment, and a thickness capable of being accommodated in the space surrounded by the cornea and the conjunctiva (palpebral conjunctiva), and is not particularly limited as long as it is suited for the curved surface of the cornea or keratoconjunctiva. It is preferable that the gel layer is a hemispheric film extending along the curved surface of the cornea or keratoconjunctiva, and is formed to have a size having a diameter of 9 to 15 mm and a thickness having a central thickness of 20 to 200 nm and a base curve of 8 to 9 mm, for example. The gel layer can also be formed into the shape of an intraocular lens such as a contact lens. The appearance is preferably transparent.

The water-soluble gelling agent contained in the keratoconjunctival cover sheet of this embodiment is an ion-responsive gelling agent. There is no particular limitation on the water-soluble gelling agent as long as it can be dissolved in the aqueous solvent, has a gelation ability in the aqueous solution in the presence of the metal ions, and is physiologically and pharmaceutically acceptable. There is no particular limitation on the type, producing method, and the like, and naturally occurring or artificially synthesized water-soluble gelling agents may be used. Commercially available water-soluble gelling agents may also be used. The term "physiologically acceptable" means that the water-soluble gelling agent has tolerable stability when applied to a living organism, and exhibits minimum toxicity against the living organism to which the water-soluble gelling agent has been applied and can thus be safely used. The term "pharmaceutically acceptable" means that the water-soluble gelling agent has tolerable stability during the preparation of an eye-drop medicine or the like.

Specific examples of the water-soluble gelling agent include, but are not limited to, anionic polysaccharides having anionic groups such as a carboxy group (-COOH), a sulfonate group (-SO₃H), and a phosphate group (-PO₄H₂) in their molecules, such as alginic acid and its derivatives, kappa (κ)-carrageenan and its derivatives, iota ( 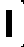 )-carrageenan and its derivatives, gellan gum such as native gellan gum and deacylated gellan gum and its derivatives, low methoxyl pectin (LM pectin) containing galacturonic acid methyl ester at a ratio of less than 50% and its derivatives, xanthan gum and its derivatives, carboxymethyl cellulose and its derivatives, and hyaluronic acid and its derivatives, and anionic macromolecules such as polyacrylic acid and its derivatives. These compounds can be used alone, in combination of two or more, or as a complex of two or more. It is known that carboxy groups, sulfonate groups, phosphate groups, and the like in the anionic macromolecules such as anionic polysaccharides dissociate a proton in an aqueous solvent and become negatively charged, and thus metal ions having generally a valency of two or more form intermolecular crosslinks, leading to gelation. Examples thereof further include cationic macromolecules such as cationic polysaccharides having cationic groups such as an amino group (-NH₂) in their molecules, such as chitosan and its derivatives.

The term "derivative" widely encompasses compounds derived from a mother compound, such as salts, esters, and ethers. Examples thereof include, but are not limited to, the above-mentioned anionic macromolecules having a structure in which at least a portion of carboxy groups, sulfonate groups, and phosphate groups is substituted by a salt, and the above-mentioned anionic macromolecules having a structure in which at least a portion of carboxy groups, sulfone groups, and phosphate groups is substituted by an ester.

Alginic acid or alginic acid derivatives can be preferably used. Alginic acid is an intercellular mucopolysaccharide derived from algae and the like, and it is known that some bacteria produce alginic acid in which some groups are substituted by acetic acid ester and secretes it to the outside of the living organism. Specific examples of alginic acid or alginic acid derivatives include: alginic acid; alkali metal salts of alginic acid such as sodium alginate and potassium alginate; alginic acid salts such as ammonium alginate; and alginic acid esters such as alginic acid propylene glycol ester. Alginic acid is a polysaccharide in which mannuronic acid (M) and guluronic acid (G) linearly polymerize, and includes a M block constituted by only M components, a G block constituted by only G components, and a random block in which the M components and the G components are randomly linked together. Carboxy groups in the molecules of alginic acids are crosslinked via polyvalent ions, and a network structure including molecular chains is formed, which results in gelation. The gelation concentration of alginic acids and the gel strength are affected by the M/G ratio or the pattern of MG arrangement. The M/G ratio of alginic acid used is preferably 0.5 to 3 or 0.5 to 2.5, but there is no particular limitation thereto. Commonly, gelation by G component-rich alginic acids (e.g., the M/G ratio is 0.5 to 1.0) can be caused by metal ions at a lower concentration, and rigid gel having a high gel strength is formed. In cases of M component-rich alginic acids (e.g., the M/G ratio is 1.0 or more or 1.5 or more), soft gel is formed. Accordingly, the M/G ratio and pattern of MG arrangement of alginic acids can be changed as appropriate depending on the gel strength, shape, biodegradability, and the like of the gel layer that are required in accordance with the purpose of the keratoconjunctival cover sheet.

Although the weight-average molecular weight, molecular weight distribution, and viscosity of the water-soluble gelling agent can be changed as appropriate depending on the type of the water-soluble gelling agent, and the gel strength, shape, biodegradability, and the like of the gel layer that are required in accordance with the purpose of the keratoconjunctival cover sheet, the water-soluble gelling agent preferably has a weight-average molecular weight of 10,000 to 300,000, and 50 to 1000 cp. Although the content of the water-soluble gelling agent can also be changed as appropriate depending on the type and molecular weight of the water-soluble gelling agent, and the gel strength, shape, biodegradability, and the like of the gel layer that are required in accordance with the purpose of the keratoconjunctival cover sheet, the content of the water-soluble gelling agent can be preferably set to 5% to 95% (concentration). The water-soluble gelling agent is used as an aqueous eye-drop solution to be applied to the eye, and therefore, its solubility in water is preferably 1 mg/g or more at 25°C.

There is no particular limitation on the metal ions contained in the keratoconjunctival cover sheet as long as the metal ions have an ability to cause gelation by the water-soluble gelling agent and are physiologically and pharmaceutically acceptable. Accordingly, the metal ions can be selected as appropriate in accordance with the water-soluble gelling agent to be used.

The metal ions are monovalent or polyvalent metal ions, and may be positive ions or negative ions. Examples of monovalent positive metal ions include, but are not limited to, alkali metal ions such as sodium ions and potassium ions, and examples of polyvalent positive metal ions include, but are not limited to, calcium ions, magnesium ions, chromium ions, manganese ions, iron ions, cobalt ions, copper ions, zinc ions, selenium ions, molybdenum ions, barium ions, and aluminum ions. These metal ions can be used alone or in combination of two or more. There is no particular limitation on the form of the positive metal ions, and the positive metal ions may be in the form of an inorganic salt such as a chloride, carbonate, or phosphate, or in the form of an organic salt such as an acetate, lactate, gluconate, formate, citrate, succinate, maleate, or ascorbate. Furthermore, the metal ions may also be hexaaquacobalt ions or the like, which are complex ions to which ligands link. Examples of negative metal ions include: polyatomic ions in which a metal ion and an oxygen atom and the like link together, such as permanganate ions, dichromate ions, and chromate ions; and complex ions in which ligands link to a metal ion, such as hexacyanoiron ions and tetrahydroxide zincate ions. These metal ions can be used alone or in combination of two or more. Positive metal ions and negative metal ions may be used in combination, thus making it possible to adjust the gelation speed, gel strength, and the like of the gel layer.

When alginic acid or its derivative, i-carrageenan or its derivative, or LM pectin or its derivative is used as the water-soluble gelling agent, polyvalent positive metal ions can be preferably used, and divalent positive metal ions such as calcium ions or magnesium ions can be particularly preferably used. Also in the case of polyacrylic acid or its derivative, polyvalent positive metal ions can be used. In the case of κ-carrageenan or its derivative, monovalent positive metal ions, and particularly preferably potassium ions or sodium ions, can be used. In the case of gellan gum or its derivative, any of the above-listed metal ions can be preferably used. In the case of xanthan gum or its derivative, gel is formed at a neutral level using trivalent positive metal ions such as aluminum ions or iron ions, and thus these trivalent positive metal ions can be used.

Although the content of metal ions can be changed as appropriate depending on the type and molecular weight of the water-soluble gelling agent, the type of metal ions, and the gel strength, shape, biodegradability, and the like of the gel layer that are required in accordance with the purpose of the keratoconjunctival cover sheet, the content of metal ions can be prepared to be 1 mg/g to 100 mg/g concentration.

As described above, the keratoconjunctival cover sheet of this embodiment is constituted by the gel layer, which is a structure that has a three-dimensional network structure in which intramolecular or intermolecular crosslinks of the water-soluble gelling agent are formed via ionic bonds with the metal ions serving as crosslinking points, and that retains an aqueous solvent that has lost its fluidity in spaces in the network structure and thus swells. For example, in the case of alginic acid or an alginic acid derivative, carboxy groups of alginic acid or alginic acid derivative intramolecularly or intermolecularly form egg box junctions due to intervention of polyvalent metal ions, and a three-dimensional network structure is thus formed.

Additives such as buffer agents, tonicity agents, preserving agents, stabilizing agents, thickening agents, and cooling agents can be added to the keratoconjunctival cover sheet of this embodiment as needed, as long as the above-mentioned functions of the water-soluble gelling agent and metal ions are not impaired.

There is no particular limitation on the buffer agents as long as the buffer agents can adjust the pH of the keratoconjunctival cover sheet of this embodiment, particularly control fluctuations in pH caused by the addition of the metal ions or the like, and are physiologically and pharmaceutically acceptable. Examples of the buffer agents include, but are not limited to, sodium citrate, sodium acetate, sodium hydrogencarbonate, boric acid, sodium hydrogenphosphate, and sodium dihydrogenphosphate.

There is no particular limitation on the tonicity agents as long as the tonicity agents can adjust the osmotic pressure of the keratoconjunctival cover sheet of this embodiment and are physiologically and pharmaceutically acceptable. Examples of the tonicity agents include, but are not limited to, potassium chloride, sodium chloride, concentrated glycerin, glucose, and D-mannitol.

There is no particular limitation on the preserving agents as long as the preserving agents can prevent contamination of the keratoconjunctival cover sheet of this embodiment with bacteria and mold and are physiologically and pharmaceutically acceptable. Examples of the preserving agents include, but are not limited to, p-hydroxybenzoates such as methyl p-hydroxybenzoate and ethyl p-hydroxybenzoate, benzyl alcohol, chlorobutanol, benzethonium chloride, and benzalkonium chloride.

Examples of the stabilizing agents include, but are not limited to, sodium citrate, sodium thiosulfate, sodium pyrosulfite, polysorbate 80, povidone, and dextran 40. Examples of the thickening agents include, but are not limited to, methyl cellulose (MC), sodium carboxymethyl cellulose (CMC-Na), and chondroitin sulfate. Examples of the cooling agents include, but are not limited to, d-camphor, dl-camphor, I-menthol, and methyl salicylate.

When the additives are added to the keratoconjunctival cover sheet of this embodiment, the contents of the additives are determined as appropriate depending on the types and purposes of additives, and the types and contents of water-soluble agent and metal ions.

Although there is no particular limitation on the pH of the gel layer of the keratoconjunctival cover sheet of this embodiment as long as the pH is within a physiologically and pharmaceutically acceptable range, the pH is preferably adjusted to be within a range of 4.0 to 9.5, and particularly preferably within a range of 4.8 to 8.5.

The keratoconjunctival cover sheet of this embodiment may include another type of gelling agent other than ion-responsive gelling agents as a thickening agent for adjusting the viscosity of the gel layer. For example, such a thickening agent can be added thereto for the purpose of improving the gelation ability of the gel layer. A stimulus-responsive gelling agent that exhibits sol-gel transition, which is a phase transition phenomenon, due to external stimuli other than ionic response can be preferably added thereto. Examples of the stimulus-responsive gelling agent include, but are not limited to, temperature-responsive polymers, pH-responsive polymers, and photoresponsive polymers, and these polymers can be used together with the ion-responsive gelling agent to impart composite responsiveness.

The temperature-responsive polymers are macromolecules that exhibit sol-gel transition at a certain temperature, and temperature-responsive polymers whose lower critical solution temperature is near a body temperature such as about 30°C can be preferably used. Such temperature-responsive polymers are in a sol state, that is, in the form of solution, at a temperature lower than the lower critical solution temperature, whereas they are insoluble in water at a temperature higher than or equal to the lower critical solution temperature and form gel. Specific examples thereof include, but are not limited to, poly N-isopropylacrylamide, poly(N-vinylisobutylamide), polyvinyl methyl ether, and polyethylene glycol-polyethylene glycol-polyethylene glycol ABA block copolymers.

The pH-responsive polymers are macromolecules that exhibit sol-gel transition at a certain pH, and pH-responsive polymers whose phase-transition pH is preferably within a range of 4.0 to 9.5 and particularly preferably 4.8 to 8.5 can be used. Specific examples thereof include, but are not limited to, anionic polymers such as polyacrylic acid and polymethacrylic acid, which are in the form of solution in an alkaline region, and cationic polymers such as polyacrylamide, which are in the form of solution in an acidic region. The gelation ability of the above-mentioned alginic acid and its derivatives is affected by pH. Moreover, the gelation ability of high methoxyl pectin (HM pectin) and its derivatives is affected by the sugar concentration and pH and thus the high methoxyl pectin (HM pectin) and its derivatives can also be favorably used as the pH-responsive polymer.

The photoresponsive polymers are macromolecules that exhibit sol-gel transition in response to photoirradiation, and photoresponsive polymers that make phase transition in response to light wavelength, intensity, irradiation time, and the like can be used. Specific examples thereof include, but are not limited to, macromolecules in which compounds such as azobenzene and spirobenzopyran that reversibly isomerize due to photoirradiation are introduced into a portion of side chains. There is no particular limitation on macromolecules serving as a main chain, but using polymers that is responsive to external stimuli such as ions, temperatures, and pH as a main chain makes it possible to impart more composite responsiveness.

The keratoconjunctival cover sheet of this embodiment can also be constructed using, instead of the above-mentioned ion-responsive gelling agent, a stimulus-responsive gelling agent such as a temperature-responsive gelling agent, a pH-responsive gelling agent, or a photoresponsive gelling agent as a main component of the gel layer.

Furthermore, an oil component can be added to the keratoconjunctival cover sheet of this embodiment as needed, as long as the above-mentioned functions of the water-soluble gelling agent and metal ions are not impaired. Adding the oil component makes it possible to suppress water evaporation from the inside of the gel layer of the keratoconjunctival cover sheet and improve water retention while favorably maintaining the functions of the keratoconjunctival cover sheet. This makes it possible to effectively suppress the induction of a dry-eye condition and the like caused by acceleration of the evaporation of the lacrimal fluid and dryness of the eye, which have been known to be induced by wearing conventional contact lenses.

It is possible to use oil components that can be generally used in ophthalmic preparations, and the oil component may be synthetic oil, plant oil, animal oil, or mineral oil. Liquid oil, which is in the form of liquid at room temperature, is preferable, and colorless and transparent liquid oil is particularly preferable. Examples of the synthetic oil include, but are not limited to, silicone oil and the like and their derivatives. Examples of the plant oil include, but are not limited to, castor oil, flaxseed oil, sesame oil, soybean oil, corn oil, turpentine oil, almond oil, cedar wood oil, coconut oil, cottonseed oil, orange oil, rapeseed oil, sunflower oil, safflower oil, olive oil, and the like and their derivatives. Examples of the animal oil include, but are not limited to, squalane oil, orange roughy oil, horse oil, mink oil, egg-yolk oil, and the like and their derivatives. Examples of the mineral oil include, but are not limited to, paraffin oil, liquid paraffin, liquid isoparaffin, and the like and their derivatives. These oil components may be used alone or in combination of two or more.

The oil component may be of an oil-in-water type in which oil droplets of the oil component disperse in water in the gel layer of the keratoconjunctival cover sheet, or a separate-layer type such as two-layer type in which the oil component forms a layer that is separate from water in the gel layer or the gel layer itself. In the case of the oil-in-water type, it is preferable that oil droplets of the oil component uniformly disperse inside the gel layer of the keratoconjunctival cover sheet of this embodiment. In the case of the separate-layer type, an oil layer made of the oil component is formed inside the gel layer of the keratoconjunctival cover sheet or outside of the gel layer. It is preferable that a coating made of the oil component is formed on a surface of the gel layer of the keratoconjunctival cover sheet that comes into contact with air, and the coating made of the oil component allows evaporation of water inside the gel layer to be particularly effectively suppressed, thus making it possible to provide a keratoconjunctival cover sheet having a particularly excellent water-retention function.

There is no particular limitation on the content of the oil component as long as the functions of the water-soluble gelling agent and metal ions are not impaired, and the content thereof is preferably 1 to 90% and particularly preferably 1 to 50% with respect to the gel layer of the keratoconjunctival cover sheet of this embodiment. In particular, in the case of the separate-layer type, a uniform oil coating is formed on the surface of the gel layer by adjusting the content of the oil component to be within such a range, and the water retention of the keratoconjunctival cover sheet of this embodiment can be effectively improved.

Moreover, a moisturizing component can be added to the keratoconjunctival cover sheet of this embodiment as needed, as long as the above-mentioned functions of the water-soluble gelling agent and metal ions are not impaired. Similarly to the case of the above-mentioned oil component, adding the moisturizing component makes it possible to suppress water evaporation from the inside of the gel layer of the keratoconjunctival cover sheet and improve water retention while favorably maintaining the functions of the keratoconjunctival cover sheet. This makes it possible to effectively suppress the induction of a dry-eye condition and the like caused by acceleration of the evaporation of the lacrimal fluid and dryness of the eye, which have been known to be induced by wearing conventional contact lenses.

It is possible to use moisturizing components that can be generally used in ophthalmic preparations, and the moisturizing component may be of a water-soluble type or a fat-soluble type. Colorless and transparent water-soluble moisturizing components are preferable. Examples of the moisturizing components include, but are not limited to: mucopolysaccharides such as hyaluronic acid, chondroitin, and heparin; proteins such as collagen and elastin; sphingolipids such as ceramide; betaines such as trimethylglycine and poly(2-methacryloyloxyethyl phospholylcholine); sterols such as cholesterol; sugar alcohols such as sorbitol and maltitol; polyhydric alcohols such as glycerin and pentylene glycol; and aliphatic alcohols such as stearyl alcohol and cetanol. These moisturizing components may be used alone or in combination of two or more.

A water-soluble moisturizing component takes the form of an aqueous solution and is contained inside the gel layer of the keratoconjunctival cover sheet of this embodiment preferably in a uniform manner. As is the case with the above-mentioned oil component, a fat-soluble moisturizing component may be of an oil-in-water type in which the moisturizing component disperses in water in the gel layer of the keratoconjunctival cover sheet, or a separate-layer type in which the moisturizing component forms a layer that is separate from water in the gel layer or the gel layer itself.

There is no particular limitation on the content of the moisturizing component as long as the functions of the water-soluble gelling agent and metal ions are not impaired, and the content thereof is preferably 1 to 90% and particularly preferably 1 to 50% with respect to the gel layer of the keratoconjunctival cover sheet of this embodiment. Adjusting the content of the moisturizing component to be within such a range makes it possible to effectively improve the water retention of the keratoconjunctival cover sheet of this embodiment.

As described above, the keratoconjunctival cover sheet of this embodiment is mostly constituted by water, and thus exhibits good oxygen-permeability and good wettability and can suppress ophthalmopathy such as dry-eye. The keratoconjunctival cover sheet of the present disclosure is highly hydrophilic and can thus suppress attachment of biological components such as lipids and proteins derived from the lacrimal fluid and eye discharge, which are oleaginous components, and cosmetic components typified by an eye makeup such as an eye shadow or a mascara and may exhibit good contamination-resistance. Furthermore, the keratoconjunctival cover sheet of the present disclosure can be applied through ocular instillation, which is a simple technique, and the gel layer can be configured to naturally biodegrade in the eye and be discharged. Therefore, the keratoconjunctival cover sheet of the present disclosure can be easily handled without troublesome work unlike a case where conventional contact lenses and the like are applied and removed. In addition, the gel layer is a soft material, and thus comfort can be achieved. Moreover, the keratoconjunctival cover sheet of the present disclosure is made of a water-soluble gelling agent and metal ions, and is thus very inexpensive. The keratoconjunctival cover sheet of the present disclosure with such properties can be favorably used for medical treatment, visual correction, a cosmetic application, or the like.

Adding the oil component, moisturizing component, or the like to the keratoconjunctival cover sheet of this embodiment makes it possible to suppress water evaporation from the inside of the gel layer of the keratoconjunctival cover sheet and improve water retention while favorably maintaining the functions of the keratoconjunctival cover sheet. This makes it possible to effectively suppress the induction of a dry-eye condition and the like caused by acceleration of the evaporation of the lacrimal fluid and dryness of the eye, which have been known to be induced by wearing conventional contact lenses.

### Mode of utilizingkeratoconjunctival cover sheet

Specific functions can be imparted to the keratoconjunctival cover sheet of this embodiment in order to favorably use the keratoconjunctival cover sheet for the purpose of medical treatment, visual correction, a cosmetic application, or the like. The keratoconjunctival cover sheet can also be used as an intraocular lens such as a contact lens. Hereinafter, modes of utilization will be described in detail, but there is no limitation thereto. The keratoconjunctival cover sheet can be used for not only a single application but also a combination of a plurality of applications.

### Keratoconjunctival cover sheet for medical treatment

A therapeutically effective amount of a drug can be added to the gel layer of the keratoconjunctival cover sheet of this embodiment, and thus a keratoconjunctival cover sheet for treatment, prevention, and diagnosis of diseases, particularly ocular diseases, can be provided. The keratoconjunctival cover sheet covers the cornea or keratoconjunctiva along the shape of the cornea or keratoconjunctiva, and therefore, the drug can be directly and efficiently delivered to the cornea or keratoconjunctiva. In addition, the drug can efficiently move through the cornea or keratoconjunctiva to the portions inside the eyeball, such as the conjunctiva, the sclera, the anterior eye chamber inside the eye, the iris, the posterior eye chamber, the ciliary body, the crystalline lens, the vitreous body and the retina, and particularly to the posterior ocular segment, and thus the therapeutic effect can be improved. The gel layer can be configured to biodegrade and to release the drug contained therein as the gel layer degrades, that is, sustained-release properties can be imparted to the gel layer. Accordingly, the drug release speed can be controlled, and thus a sustained-release preparation of the drug can be produced. With the sustained-release preparation of the drug, the drug at an appropriate drug concentration can be continuously released from the keratoconjunctival cover sheet for a certain period of time, and thus the bioavailability of the drug can be improved. Furthermore, the frequency of administration can be reduced, and therefore, side-effects caused by overdosage, such as a systemic side-effect caused by absorption of the drug through the mucosa of the lacrimal sac, can also be reduced, and the medication compliance can also be improved.

There is no particular limitation on the ocular diseases, but the keratoconjunctival cover sheet of this embodiment can be preferably used for diseases whose progress or onset can be effectively suppressed by continuous or topical administration of the drug. Examples thereof include, but are not limited to: corneal diseases such as cornea infections; diseases of the retina and vitreous body such as retinitis pigmentosa, age-related macular degeneration, diabetic retinopathy, retinal vein occlusion, and central serous chorioretinopathy; diseases of the crystalline lens such as glaucoma and cataract; diseases of the uvea such as uveitis and infectious endophthalmitis; conjunctival diseases such as allergic conjunctivitis and viral conjunctivitis; and dry eye.

There is no particular limitation on the drug as long as the drug can be used for treatment, prevention, and diagnosis of diseases, particularly ocular diseases. Examples of the drug include, but are not limited to, antibacterial drugs such as levofloxacin, ofloxacin, and chloramphenicol; antibiotics such as cefmenoxime hemihydrochloride; antiviral drugs such as aciclovir; antifungal drugs such as pimaricin; anti-inflammatory drugs such as diclofenac sodium and azulen sodium sulfonate; anti-allergic drugs such as disodium cromoglycate and ketotifen fumarate; adrenocortical steroid drugs such as prednisolone and fluorometholone; therapeutic drugs for the cornea such as sodium hyaluronate and sodium chondroitin sulfate; therapeutic drugs for glaucoma such as carteolol and timolol; mydriatic drugs such as tropicamide; topical anesthetic drugs such as oxybuprocaine; anti-cataract drugs such as pirenoxine; therapeutic drugs for retinitis pigmentosa such as carbonic anhydrase inhibitors (e.g., dorzolamide); therapeutic drugs for age-related macular degeneration such as anti-VEGF antibodies (e.g., ranibizumab), VEGF inhibitors (e.g., aflibercept), and verteporphyrin; therapeutic drugs for diabetic retinopathy such as VEGF inhibitors and steroid drugs (e.g., triamcinolon); mucin secretagogues such as rebamipide and diquafosol; therapeutic drugs for dry eye such as hyaluronic acid, cyclosporine, and artificial lacrimal fluid; and vitamin drugs.

There is no particular limitation on the form of the drug as long as the drug can be enclosed in the gel layer, and the drug may be in the form of a solid or a liquid. In the case of a solid, it is preferable that the drug is dissolved or suspended in an appropriate aqueous solvent when applied to the eye. The drug may also be prepared in the form of nanoparticles. When prepared in the form of nanoparticles, substances that are poorly soluble in water can be dispersed in an aqueous solvent. When nanoparticles of the drug are enclosed in the gel layer, the drug can be uniformly dispersed in the gel layer, thus making it possible to further improve the sustained drug release properties. Accordingly, the drug can be more efficiently delivered, and thus the drug can be more efficiently delivered to the posterior ocular segment including the vitreous body, retina, and the like. The nanoparticles herein are particles having a size of the order of nanometers (about 1 nm to less than 1000 nm), and can be prepared using a method known in the art. The nanoparticles can be prepared using physical methods such as vapor deposition, laser ablation, homogenization, sonication, and crushing, and chemical methods such as a sol-gel method, a micelle method, a precipitation method, a spray dry method, a liposome method, and an emulsion method, for example, but there is no limitation thereto.

There is no particular limitation on the content of the drug as long as the drug can be enclosed in the gel layer. The content of the drug in the gel layer is preferably 0.001 to 10 wt%, and can be determined as appropriate by a person skilled in the art depending on the therapeutically effective amount and solubility of the drug, and a period of time for which the drug is being slowly released.

Furthermore, the keratoconjunctival cover sheet of this embodiment can be used to protect the cornea or keratoconjunctiva from the external environment including external stimuli such as light (e.g., ultraviolet rays), temperatures, viruses, bacteria, and antigenic substances. For example, the keratoconjunctival cover sheet can suppress attachment of antigenic substances (allergens) such as airborne pollen, mites, house dust, and animal hair and dirt that induce an allergic reaction in the cornea or keratoconjunctiva, and prevent the onset of allergic diseases such as allergic conjunctivitis. The keratoconjunctival cover sheet can be used to protect a damaged cornea or keratoconjunctiva or heal a wound. For example, the keratoconjunctival cover sheet can be used for regenerative treatment, prevention of postoperative infection, and the like of a cornea or keratoconjunctiva. When the eye cannot be closed due to facial palsy (i.e., lagophthalmos), the eyeball gets dry. This may cause ocular inflammation, superficial punctate keratopathy, opacity, ulcer, bacterial or fungal infectious diseases, and the like, and the conditions may be aggravated. The keratoconjunctival cover sheet of this embodiment can be used to prevent dryness of the surface of the eye caused by lagophthalmos involved in facial palsy that occurs in everyday life or before, during, or after an operation.

### Keratoconjunctival cover sheet for visual correction

The thickness and the like of the keratoconjunctival cover sheet of this embodiment can be adjusted by varying the molecular weight, type, and concentration of the gelling agent included in the keratoconjunctival cover sheet to control the viscosity of the gel layer, thus making it possible to vary the light refractive index of the keratoconjunctival cover sheet.

A water-soluble component with a high refractive index can also be added to the gel layer, thus making it possible to vary the light refractive index of the keratoconjunctival cover sheet of this embodiment. For example, the refractive index of an aqueous solution of sucrose varies from 1.33 to a little over 1.5 with the sucrose concentration. Similarly to this case, the refractive index of an aqueous solution varies with the concentration of the water-soluble component with a high refractive index. The gel layer constituting the keratoconjunctival cover sheet is mostly constituted by water, and therefore, the refractive index of the keratoconjunctival cover sheet can be controlled using such properties.

When the refractive index of an aqueous solution of a water-soluble component or the refractive index of a water-soluble component in the form of liquid is preferably 1.5 or more and particularly preferably 1.9 or more, the water-soluble component is referred to as "water-soluble component with a high refractive index". There is no particular limitation on the water-soluble component with a high refractive index as long as it is physiologically and pharmaceutically acceptable. It is preferable that the refractive index is determined through measurement performed with an Abbe's refractometer using a D-line of the sodium spectrum as a ray of light at a temperature of 20°C, preferably in accordance with the Japanese Pharmacopoeia.

Examples of the water-soluble component with a high refractive index include sugars, polyhydric alcohols, and water-soluble polymers. Examples of the sugars include: water-soluble monosaccharides such as glucose, sorbitose, mannose, galactose, fructose, xylose, and arabinose; water-soluble polysaccharides such as water-soluble disaccharides (e.g., sucrose, lactose, maltose, and trehalose); amino sugars such as glucosamine; and derivatives thereof, and sucrose can be preferably used. The polyhydric alcohols have two or more hydroxy groups in their molecules, and examples thereof include: glycols such as ethylene glycol, propylene glycol, butylene glycol, pentylene glycol, and diethylene glycol; glycerins such as glycerin and diglycerin; sugar alcohols such as sorbitol, maltitol, mannitol, xylitol, arabitol, and lactitol; and derivatives thereof. Examples of the water-soluble polymers include polyvinyl alcohol, polyvinyl pyrrolidone, carboxyvinyl polymers, hydroxypropyl cellulose, carboxymethyl cellulose, carboxymethylethyl cellulose, polyethylene glycol (polyethylene oxide), polypropylene glycol (polypropylene oxide), acrylic polymers, maleic polymers, and derivatives thereof. The above-mentioned stimulus-responsive gelling agents can also be used as the water-soluble polymer, and this makes it possible to improve the gelation ability as well as control the refractive index. The water-soluble components with a high refractive index can be used alone or as a mixture of two or more. When a compound that is in a solid state at room temperature, such as sucrose or glucose, is used, it is preferable that the compound is dissolved in an appropriate aqueous solvent such as water, purified water, or a physiological saline solution to prepare a aqueous solution. Since the refractive index of an aqueous solution varies with the concentration of a medium, the concentration is adjusted as appropriate to be an appropriate concentration.

Some water-soluble components with a high refractive index have a function of the above-mentioned moisturizing components. In such a case, a function of controlling the refractive index and a function of improving water retention can be imparted at the same time.

The keratoconjunctival cover sheet of this embodiment can be formed as a laminate of a plurality of gel layers, and the light refractive index varies due to the interface formed between the gel layers, thus making it possible to control the refractive index. At this time, the keratoconjunctival cover sheet may be a laminate obtained by laminating gel layers including a single component that is a combination of a single type of water-soluble gelling agent and a single type of metal ion, or a laminate obtained by laminating gel layers including a plurality of components that are different combinations of water-soluble gelling agents and metal ions.

Furthermore, the refractive index of the keratoconjunctival cover sheet of this embodiment can also be controlled by adjusting the factors of the gel layer associated with the refractive index such as the viscosity, magnetic permeability, dielectric constant, electric conductivity, and gel thickness. The viscosity of the gel layer can be adjusted according to the type, molecular weight, concentration, and the like of the water-soluble gelling agent, for example. The magnetic permeability, dielectric constant, and electric conductivity of the gel layer can be adjusted according to the type, molecular weight, concentration, and the like of the water-soluble gelling agent, for example, particularly according to the type of charged functional groups of the water-soluble gelling agent, a ratio of free charged functional groups of the water-soluble gelling agent that are not linked to the metal ions, the type and concentration of the metal ions, and the like, particularly according to the valency and ionization energy of the metal ions, the type of sugar, polyhydric alcohol, water-soluble polymer, and the like that are added as a water-soluble component with a high refractive index, and the like. The gel thickness of the gel layer can be adjusted according to the type, molecular weight, concentration, amount, and the like of the water-soluble gelling agent, for example. When a plurality of gel layers are laminated, laminating gel layers that differ in the physical properties such as the viscosity, magnetic permeability, dielectric constant, electric conductivity, and gel thickness makes it possible to achieve control of various light refractive indices.

In this manner, the keratoconjunctival cover sheet can be used as a soft contact lens for myopia correction, hyperopia correction, myopia-hyperopia correction, and the like, and adjusting the lamination of gel layers, the type, molecular weight, and concentration of the water-soluble gelling agent, the type and concentration of the metal ions, the type and concentration of the water-soluble component with a high refractive index, and the like makes it possible to provide tailor-made contact lenses optimized for individual users.

### Keratoconjunctival cover sheet for cosmetic application

A coloring agent can be added to the gel layer of the keratoconjunctival cover sheet of this embodiment, thus making it possible to color the keratoconjunctival cover sheet. In this manner, the keratoconjunctival cover sheet can be used for a cosmetic application, such as color contact lenses.

There is no particular limitation on the coloring agent as long as it can color the gel layer and is physiologically and pharmaceutically acceptable. Accordingly, the coloring agent may be derived from a synthetic colorant or a natural colorant. Colorants that are acceptable as food additives can be preferably used, and water-soluble colorants are preferable. Specific examples of the synthetic colorants include, but are not limited to, tar-based colorants such as Red No. 2, Red No. 3, Red No. 40, Red No. 102, Red No. 104, Red No. 105, Red No. 106, Yellow No. 4, Yellow No. 5, Blue No. 1, Blue No. 2, and Green No. 3. Examples of the natural colorants include, but are not limited to, carotenoid-based colorants such as an annatto colorant, a gardenia yellow colorant, and crocin, quinone-based colorants, anthocyanin-based colorants, flavonoid-based colorants, and porphyrin-based colorants. These colorants can be used alone or in combination of two or more.

When the keratoconjunctival cover sheet of this embodiment is used as a color contact lens, it is preferable that a concentric annular colored region is formed in a region located outside the central region. For example, it is preferable that an annular colored region is formed on a surface of the keratoconjunctival cover sheet that comes into contact with the cornea or keratoconjunctiva, along the outer circumference of the cornea or along a region located outside of the outer circumference of the cornea.

### Method for producing keratoconjunctival cover sheet

A method for producing a keratoconjunctival cover sheet of this embodiment is a method for producing the keratoconjunctival cover sheet of this embodiment having the above-mentioned properties, and includes a step of applying a water-soluble gelling agent to an eye in which a water-soluble gelling agent exhibiting a gelation ability in the presence of metal ions is applied to an anterior ocular segment, and a step of applying metal ions to an eye in which the metal ions are applied to the anterior ocular segment, wherein a keratoconjunctival cover sheet is produced by forming a gel layer on a cornea or keratoconjunctiva through gelation by the water-soluble gelling agent caused by the metal ions forming crosslinks.

The method for producing a keratoconjunctival cover sheet of this embodiment can be configured such that the step of applying a water-soluble gelling agent to an eye in which a water-soluble gelling agent exhibiting a gelation ability in the presence of metal ions is applied to an anterior ocular segment is taken as a first ocular instillation step, the step of applying metal ions to an eye in which the metal ions are applied to the anterior ocular segment is taken as a second ocular instillation step, and the metal ions are applied onto the water-soluble gelling agent applied to the eye in the first ocular instillation step to form a gel layer on a cornea or keratoconjunctiva through gelation by the water-soluble gelling agent ((A) described below). The method for producing a keratoconjunctival cover sheet of this embodiment can also be configured such that the step of applying metal ions to an eye in which metal ions are applied to an anterior ocular segment is taken as a second ocular instillation step, the step of applying a water-soluble gelling agent to an eye in which a water-soluble gelling agent exhibiting a gelation ability in the presence of the metal ions is applied to the anterior ocular segment is taken as a second ocular instillation step, and the water-soluble gelling agent is applied onto the metal ions applied to the eye in the first ocular instillation step to form a gel layer on a cornea or keratoconjunctiva through gelation by the water-soluble gelling agent ((B) described below). Hereinafter, the steps will be described in detail.

### (A) Case where first ocular instillation step is step of applying water-soluble gelling agent to eye and second ocular instillation step is step of applying metal ions to eye

### (i) Step of applying water-soluble gelling agent to eye (first ocular instillation step)

In the step of applying a water-soluble gelling agent to an eye, it is preferable to apply an aqueous solution of a water-soluble gelling agent obtained by dissolving the water-soluble gelling agent in an appropriate aqueous solvent to the anterior ocular segment. Examples of the aqueous solvent include water, purified water, and a physiological saline solution, and additives such as a buffer agent, a tonicity agent, a preserving agent, a stabilizing agent, an emulsifying agent, a thickening agent, and a cooling agent can be added as needed. Although there is no particular limitation on the pH as long as the pH is within a physiologically and pharmaceutically acceptable range, the pH is preferably adjusted to be within a range of 4.0 to 9.5, and particularly preferably within a range of 4.8 to 8.5, because gelation by the water-soluble gelling agent occurs at a low pH in some cases. A configuration in which the water-soluble gelling agent is dissolved or diluted just before use so as to be capable of being prepared just before use is also possible.

The water-soluble gelling agent is applied to the anterior ocular segment, preferably onto the cornea, through ocular instillation. The concentration of the water-soluble gelling agent can be changed as appropriate according to the type, molecular weight, and ocular instillation amount of the water-soluble gelling agent, and the gel strength, shape, biodegradability, and the like required in accordance with the purpose of the keratoconjunctival cover sheet, but is set to 5 to 95%. The ocular instillation amount can be changed as appropriate, but is preferably 10 µL to 200 µL, which can be applied to the eye in one instance, or over multiple instances. The form of ocular instillation can be changed as appropriate, and ocular instillation can be performed through dripping or spraying. After ocular instillation, eyelid opening-closing movement such as blinking and eye movement is allowable, but it is preferable to not perform blinking or the like. Also, there is no particular limitation on the posture of the user during ocular instillation, and a lying posture, seated posture, and upright position are allowable.

In the step of applying a water-soluble gelling agent to an eye, ocular instillation may be performed using a unit-dose type eye-drop solution for a single use or a multiple-dose type eye-drop solution that can be repeatedly used.

### (ii) Step of applying metal ions to eye (second ocular instillation step)

In the step of applying metal ions to an eye, it is preferable to apply an aqueous solution of metal ions obtained by dissolving the metal ions in an appropriate aqueous solvent to the anterior ocular segment. Examples of the aqueous solvent include water, purified water, and a physiological saline solution, and additives such as a buffer agent, a tonicity agent, a preserving agent, a stabilizing agent, an emulsifying agent, a thickening agent, and a cooling agent can be added as needed. Although there is no particular limitation on the pH as long as the pH is within a physiologically and pharmaceutically acceptable range, the pH is preferably adjusted to be within a range of 4.0 to 9.5, and particularly preferably within a range of 4.8 to 8.5. A configuration in which the metal ions are dissolved or diluted just before use so as to be capable of being prepared just before use is also possible.

The metal ions are applied to the anterior ocular segment, preferably onto the cornea, through ocular instillation. The metal ions are applied to the eye so as to come into contact with the aqueous gelling agent applied to the eye in the step of applying a water-soluble gelling agent to an eye. The concentration of the metal ions can be changed as appropriate according to the type, molecular weight, concentration, ocular instillation amount, and the like of the water-soluble gelling agent, and the gel strength, shape, biodegradability, and the like required in accordance with the purpose of the keratoconjunctival cover sheet, but is preferably set to 1 mg/g to 100 mg/g. The ocular instillation amount can be changed as appropriate, but is preferably 10 µL to 200 µL, which can be applied to the eye in one instance, or over multiple instances. The form of ocular instillation can be changed as appropriate, and ocular instillation can be performed through dripping or spraying. The interval between the step of applying a water-soluble gelling agent to an eye and the step of applying metal ions to an eye is preferably about 30 seconds. After ocular instillation, eyelid opening-closing movement such as blinking and eye movement is allowable, but it is preferable to not perform blinking or the like. Also, there is no particular limitation on the posture of the user during ocular instillation, and a lying posture, seated posture, and upright position are allowable.

In the step of applying metal ions to an eye, ocular instillation may be performed using a unit-dose type eye-drop solution for a single use or a multiple-dose type eye-drop solution that can be repeatedly used.

Soon after the step of applying metal ions to an eye, a gel layer is formed through gelation by the water-soluble gelling agent caused by the metal ions forming crosslinks, and a keratoconjunctival cover sheet is thus produced. During gelation, the aqueous solution of the water-soluble gelling agent dripped onto the cornea gelates from a surface portion that comes into contact with the metal ions to the inside through ion-crosslinking. At the same time, the aqueous solution of the water-soluble gelling agent maintains its shape due to the surface tension even after it comes into contact with the metal ions. Accordingly, the resultant gel has the same shape as that when the water-soluble gelling agent was dripped, and therefore, a gel layer extending along the shape of the cornea or keratoconjunctiva, which is the site on which the water-soluble gelling agent was dripped, can be formed.

### (B) Case where first ocular instillation step is step of applying metal ions to eye and second ocular instillation step is step of applying water-soluble gelling agent to eye

### (i) Step of applying metal ions to eye (first ocular instillation step)

In the step of applying metal ions to an eye, it is preferable to apply an aqueous solution of metal ions obtained by dissolving the metal ions in an appropriate aqueous solvent to the anterior ocular segment. The form of ocular instillation, the ocular instillation amount, and the like in the step of applying metal ions to an eye can be set according to (A) above.

### (ii) Step of applying water-soluble gelling agent to eye (second ocular instillation step)

In the step of applying a water-soluble gelling agent to an eye, it is preferable to apply an aqueous solution of a water-soluble gelling agent obtained by dissolving the water-soluble gelling agent in an appropriate aqueous solvent to the anterior ocular segment. The water-soluble gelling agent is applied to the eye so as to come into contact with the metal ions applied to the eye in the step of applying metal ions to an eye. The form of ocular instillation, the ocular instillation amount, and the like in the step of applying a water-soluble gelling agent to an eye can be set according to (A) above.

In the same manner as in (A) above, soon after the step of applying a water-soluble gelling agent to an eye, a gel layer is formed through gelation by the water-soluble gelling agent caused by the previously applied metal ions forming crosslinks, and a keratoconjunctival cover sheet is thus produced.

### (C) Additional steps

The method for producing a keratoconjunctival cover sheet of this embodiment can be provided with a step of adding an oil component, moisturizing component, or the like to the keratoconjunctival cover sheet of this embodiment as needed in addition to the step of applying a water-soluble gelling agent to an eye and the step of applying metal ions to an eye.

A step of applying an oil component to an eye in which an oil component is applied to the anterior ocular segment can be preferably provided. There is no particular limitation on the form of ocular instillation, ocular instillation amount, and the like in the application of an oil component to an eye as long as the above-mentioned functions of the water-soluble gelling agent and metal ions are not impaired. For example, an oil component can be applied to the eye together with the aqueous solution of the water-soluble gelling agent in the step of applying a water-soluble gelling agent to an eye, or together with the aqueous solution of the metal ions in the step of applying metal ions to an eye. The step of applying an oil component to an eye may also be performed independently from the step of applying a water-soluble gelling agent to an eye and the step of applying metal ions to an eye.

When the oil component is applied to the eye together with the aqueous solution of the water-soluble gelling agent, an oil-in-water type eye-drop solution in which oil droplets of the oil component are dispersed in the aqueous solution of the water-soluble gelling agent may be applied to the eye, or a separate-layer type eye-drop solution in which the oil component forms a layer that is separate from the water-soluble gelling agent may be applied to the eye. When the oil component is applied to the eye using the oil-in-water type eye-drop solution including the oil component and the aqueous solution of the water-soluble gelling agent in the step of applying a water-soluble gelling agent to an eye, a keratoconjunctival cover sheet in which the oil droplets of the oil component are dispersed in the gel layer is formed by the metal ions forming crosslinks. When the oil component is applied to the eye using the separate-layer type eye-drop solution, an oil layer, which generally has a smaller specific gravity, is formed as an upper layer, that is, an oil layer made of the oil component is formed on a surface of the keratoconjunctival cover sheet that comes into contact with air, because ocular instillation means dripping a drug solution onto the anterior ocular segment. Accordingly, a keratoconjunctival cover sheet in which an oil coating is formed on a surface of the gel layer that comes into contact with air is formed by the metal ions forming crosslinks. When the oil component is applied to the eye independently from the step of applying a water-soluble gelling agent to an eye and the step of applying metal ions to an eye, it is preferable to continuously apply the oil component to the eye before and after the water-soluble gelling agent or metal ions are applied to the eye. Moreover, the oil component may also be applied onto the gel layer formed by the metal ions forming crosslinks of the water-soluble gelling agent such that a coating made of the oil component is formed thereon.

When the oil component is applied to the eye together with the water-soluble gelling agent in the step of applying a water-soluble gelling agent to an eye, it is preferable that the method for producing a keratoconjunctival cover sheet of this embodiment is performed in accordance with the procedure described in (B) above, that is, the procedure in which the first ocular instillation step is the step of applying metal ions to an eye and the second ocular instillation step is the step of applying a water-soluble gelling agent to an eye, but the procedure described in (A) above is also possible. In the case of the procedure described in (B) above, gelation occurs soon after the water-soluble gelling agent, which has a larger specific gravity, efficiently comes into contact with the previously applied metal ions on the keratoconjunctiva and the gel layer is thus formed, while the oil component, which has a smaller specific gravity, can form a coating that uniformly covers the upper surface of the gel layer, that is, a surface of the gel layer that comes into contact with air, thus making it possible to effectively suppress water evaporation from the inside of the gel layer.

There is no particular limitation on the addition amount of the oil component as long as the functions of the water-soluble gelling agent and metal ions are not impaired, and the addition amount thereof is preferably 1 to 90%, and particularly preferably 1 to 50%, with respect to the aqueous solution of the water-soluble gelling agent.

In the step of applying an oil component to an eye, ocular instillation may be performed using a unit-dose type eye-drop solution for a single use or a multiple-dose type eye-drop solution that can be repeatedly used. When the oil component is applied to the eye in addition to the aqueous solution of the water-soluble gelling agent, it is preferable to apply, to the eye, a unit-dose type eye-drop solution obtained by mixing the oil component and the aqueous solution of the water-soluble gelling agent, and this makes it possible to favorably adjust the ocular instillation amounts and ocular instillation ratio of the water-soluble gelling agent and the oil component.

A step of applying a moisturizing component to an eye, in which a moisturizing component is applied to the anterior ocular segment, can be preferably provided. There is no particular limitation on the form of ocular instillation, ocular instillation amount, and the like in the application of a moisturizing component to an eye as long as the above-mentioned functions of the water-soluble gelling agent and metal ions are not impaired. For example, a moisturizing component can be applied to the eye together with the aqueous solution of the water-soluble gelling agent in the step of applying a water-soluble gelling agent to an eye, or together with the aqueous solution of the metal ions in the step of applying metal ions to an eye. The step of applying a moisturizing component to an eye may also be performed independently from the step of applying a water-soluble gelling agent to an eye and the step of applying metal ions to an eye.

When a water-soluble moisturizing component is applied to the eye together with the aqueous solution of the water-soluble gelling agent, for example, a solution obtained by dissolving the moisturizing component in the aqueous solution of the water-soluble gelling agent can be applied to the eye. In a case of a fat-soluble moisturizing component, as is the case with the above-mentioned oil component, an oil-in-water type eye-drop solution in which oil droplets of the moisturizing component are dispersed may be applied to the eye, or a separate-layer type eye-drop solution in which the moisturizing component forms a layer that is separate from the water-soluble gelling agent may be applied to the eye.

There is no particular limitation on the addition amount of the moisturizing component as long as the functions of the water-soluble gelling agent and metal ions are not impaired, and the addition amount thereof is preferably 1 to 90%, and particularly preferably 1 to 50%, with respect to the aqueous solution of the water-soluble gelling agent.

In the step of applying a moisturizing component to an eye, ocular instillation may be performed using a unit-dose type eye-drop solution for a single use or a multiple-dose type eye-drop solution that can be repeatedly used. When the moisturizing component is applied to the eye in addition to the aqueous solution of the water-soluble gelling agent, it is preferable to apply, to the eye, a unit-dose type eye-drop solution obtained by mixing the moisturizing component and the aqueous solution of the water-soluble gelling agent, and this makes it possible to favorably adjust the ocular instillation amounts and ocular instillation ratio of the water-soluble gelling agent and the oil component.

As described above, the method for producing a keratoconjunctival cover sheet of this embodiment uses ocular instillation, which is a simple technique, and does not require technical skills and the like. The method also does not require a large-scale production apparatus or the like, and thus a keratoconjunctival cover sheet can be provided at very low cost. With the method for producing a keratoconjunctival cover sheet of the present disclosure, the application by a subject is simultaneous with the production, and the application site is used as a mold, thus making it possible to provide a tailor-made keratoconjunctival cover sheet suited for the ocular structure (particularly the shapes of the cornea and the palpebral conjunctiva) of the user.

Furthermore, with the method for producing a keratoconjunctival cover sheet of this embodiment, a sufficient amount of the metal ions, which is larger than the amount of metal ions contained in the lacrimal fluid, is added. Accordingly, gelation by the water-soluble gelling agent is thereby caused, and therefore, for example, compared with the above-mentioned cases disclosed in Patent Document 1, Patent Document 2, Non-Patent Document 1, and the like in which the gelation by a water-soluble gelling agent is caused by the lacrimal fluid, effects of increasing the gel strength, increasing the gel thickness, securing a cover area with respect to the cornea or keratoconjunctiva, and the like can be expected. Moreover, regarding the gel layer formed through the addition of the metal ions subsequent to the addition of the water-soluble gelling agent on the cornea or keratoconjunctiva as described in (A) above, it is expected that a portion of the gel on a side on which the metal ions have been added, which comes into contact with air, will be firm and hard and suppress moisture evaporation, and a portion of the gel that comes into contact with the cornea or keratoconjunctiva will be soft and have improved comfort. In addition, the gel can be formed soon after the water-soluble gelling agent is dripped, and can thus favorably cover the cornea or keratoconjunctiva, unlike the above-mentioned cases disclosed in Patent Document 1, Patent Document 2, and Non-Patent Document 1 in which a water-soluble gelling agent is involved in gelation in the conjunctival sac of the lower eyelid where the lacrimal fluid accumulates, and the resulting gel stays there.

With the method for producing a keratoconjunctival cover sheet of this embodiment provided with the step of applying an oil component or moisturizing component to an eye, a keratoconjunctival cover sheet in which moisture evaporation from the inside of the gel layer is suppressed and water retention is improved while the functions are favorably maintained can be provided. Accordingly, a keratoconjunctival cover sheet can be provided that can effectively suppress the induction of a dry-eye condition and the like caused by acceleration of the evaporation of the lacrimal fluid and dryness of the eye, which have been known to be induced by wearing conventional contact lenses.

### Mode of utilizing method for producing keratoconjunctival cover sheet

The method for producing a keratoconjunctival cover sheet of this embodiment can include a step of imparting specific functions to the keratoconjunctival cover sheet that enables the keratoconjunctival cover sheet to be favorably used for the purpose of medical treatment, visual correction, a cosmetic application, or the like. For example, the method can include a step of applying a drug, water-soluble component with a high refractive index, coloring agent, or the like to an eye. When applied to the eye, a drug, water-soluble component with a high refractive index, coloring agent, or the like is preferably used dissolved in an aqueous solvent. Examples of the aqueous solvent include water, purified water, and physiological saline solution. Additives such as a buffer agent, a tonicity agent, a preserving agent, a stabilizing agent, an emulsifying agent, a thickening agent, and a cooling agent can be added as needed. Although there is no particular limitation on the pH as long as the pH is within a physiologically and pharmaceutically acceptable range, the pH is preferably adjusted to be within a range of 4.0 to 9.5, and particularly preferably within a range of 4.8 to 8.5. A configuration in which a drug, water-soluble component with a high refractive index, coloring agent, or the like is dissolved or diluted just before use so as to be capable of being prepared just before use is also possible.

The step of applying a drug, water-soluble component with a high refractive index, or coloring agent to an eye can be performed before, simultaneously with, or after the step of applying a water-soluble gelling agent to an eye. When this step is performed simultaneously with the step of applying a water-soluble gelling agent to an eye, a single solution obtained by dissolving a drug, water-soluble component with a high refractive index, or coloring agent together with the water-soluble gelling agent in an appropriate aqueous solvent can be applied to the eye. When performed after the step of applying a water-soluble gelling agent to an eye, the step of applying a drug, water-soluble component with a high refractive index, or coloring agent to an eye is preferably performed before the step of applying metal ions to an eye. This makes it possible to sufficiently mix the drug, water-soluble component with a high refractive index, or coloring agent with the water-soluble gelling agent in the aqueous solvent before gelation caused by the metal ions, and enclose the drug, water-soluble component with a high refractive index, or coloring agent inside the gel layer, and thus the drug, water-soluble component with a high refractive index, or coloring agent can effectively exhibit its function. A configuration in which an aqueous solution of the drug, water-soluble component with a high refractive index, or coloring agent and an aqueous solution of the water-soluble gelling agent are filled into an ocular instillation container designed such that a partition or the like provided thereinside prevents these aqueous solutions from mixing together, and these aqueous solutions are mixed when applied to the eye is also possible. However, the eye-drop solution of the drug, water-soluble component with a high refractive index, or coloring agent moves relatively easily to the inside of the gel layer because the gel layer has a three-dimensional network structure, and therefore, a case where the eye-drop solution thereof is applied to the eye after gelation by the water-soluble gelling agent caused by the metal ions is not eliminated.

A region containing a drug, water-soluble component with a high refractive index, or coloring agent can be formed in only a specific region of the keratoconjunctival cover sheet of this embodiment. For example, an aqueous solution of a mixture of the drug, water-soluble component with a high refractive index, or coloring agent and the water-soluble gelling agent, and an aqueous solution of only the water-soluble gelling agent are filled into the above-mentioned ocular instillation container designed such that a partition provided thereinside prevents these aqueous solutions from mixing together, an ocular instillation nozzle thereof being also provided with a partition or the like. Since the gelation by a water-soluble gelling agent occurs instantly in the presence of the metal ions, gel is formed in a state in which these aqueous solutions maintain their shape when dripped and are not excessively mixed together, thus making it possible to form the region containing the drug, water-soluble component with a high refractive index, or coloring agent in a specific region. In the same manner, a portion containing the drug, water-soluble component with a high refractive index, or coloring agent at a different concentration can be formed in a specific region of the keratoconjunctival cover sheet of this embodiment. An example of the shape of the ocular instillation nozzle is the shape of an ocular instillation nozzle formed such that the central region and a concentric annular outer region are separated from each other. In particular, such an ocular instillation nozzle can be used to produce color contact lenses, and an annular colored region can be formed on a surface of the keratoconjunctival cover sheet that comes into contact with the cornea or keratoconjunctiva, along the outer circumference of the cornea or along a region located outside of the outer circumference of the cornea.

With the method for producing a keratoconjunctival cover sheet of this embodiment, gel layers can be laminated by repeating the step of applying a water-soluble gelling agent to an eye and the application of metal ions to the eye. The light refractive index varies due to the interface formed between the gel layers. This makes it possible to appropriately control the refractive index of the produced keratoconjunctival cover sheet. At this time, the keratoconjunctival cover sheet may be a laminate obtained by laminating gel layers including a single component that is a combination of a single type of water-soluble gelling agent and a single type of metal ion, or a laminate obtained by laminating gel layers including a plurality of components that are different combinations of water-soluble gelling agents and metal ions.

Furthermore, with the method for producing a keratoconjunctival cover sheet of this embodiment, it is possible to adjust the factors of the gel layer associated with the refractive index such as the viscosity, magnetic permeability, dielectric constant, electric conductivity, and gel thickness, thus making it possible to control the refractive index of the produced keratoconjunctival cover sheet. When a plurality of gel layers are laminated, laminating gel layers that differ in the physical properties such as the viscosity, magnetic permeability, dielectric constant, electric conductivity, and gel thickness makes it possible to achieve control of various light refractive indices.

In this manner, with the method for producing a keratoconjunctival cover sheet of this embodiment, a keratoconjunctival cover sheet that can be used as a soft contact lens for myopia correction, hyperopia correction, myopia-hyperopia correction, and the like can be provided, and adjusting the lamination of gel layers, the type, molecular weight, and concentration of the water-soluble gelling agent, the type and concentration of the metal ions, the type and concentration of the water-soluble component with a high refractive index, and the like makes it possible to provide tailor-made contact lenses optimized for individual users.

### Kit for producing keratoconjunctival cover sheet

A kit for producing a keratoconjunctival cover sheet of this embodiment is a kit for producing the keratoconjunctival cover sheet of this embodiment having the above-mentioned properties on the keratoconjunctiva, and includes an eye-drop solution of a water-soluble gelling agent obtained by dissolving a water-soluble gelling agent exhibiting a gelation ability in the presence of metal ions in an appropriate aqueous solvent such as water, purified water, or a physiological saline solution, and an eye-drop solution of metal ions obtained by dissolving the metal ions in an appropriate aqueous solvent such as water, purified water, or a physiological saline solution, and the eye-drop solution of a water-soluble gelling agent and the eye-drop solution of metal ions are filled into appropriate containers such as separate ocular instillation bottles. Additives such as a buffer agent, a tonicity agent, a preserving agent, a stabilizing agent an emulsifying agent, a thickening agent, and a cooling agent may be added to the eye-drop solution of a water-soluble gelling agent and the eye-drop solution of metal ions as needed. The water-soluble gelling agent for the eye-drop solution of a water-soluble gelling agent and the metal ions for the eye-drop solution of metal ions may be filled into containers in the form of a solid or a concentrate so as to be capable of being prepared just before use, and an appropriate separate container filled with an appropriate aqueous solvent such as water, purified water, or a physiological saline solution to be used for dissolution or dilution for the preparation just before use may be provided. Furthermore, an instruction manual of ocular instillation for users may be provided.

The eye-drop solutions of the water-soluble gelling agent and the metal ions may be accommodated in a unit-dose type ocular instillation container for a single use or a multiple-dose type ocular instillation container that can be repeatedly used. A configuration in which one of the eye-drop solutions is accommodated in a unit-dose type ocular instillation container and the other is accommodated in a multiple-dose type ocular instillation container is also possible.

The kit for producing a keratoconjunctival cover sheet of this embodiment may include an eye-drop solution containing an oil component, moisturizing component, or the like. The oil component or moisturizing component may be contained in the eye-drop solution of a water-soluble gelling agent or the eye-drop solution of metal ions, or in a separate eye-drop solution. When the oil component, moisturizing component, or the like is added to the eye-drop solution of a water-soluble gelling agent, it is preferable that the eye-drop solution is accommodated in a unit-dose type ocular instillation container, and this makes it possible to favorably adjust the ocular instillation amounts and ocular instillation ratio of the water-soluble gelling agent and the oil component or moisturizing component.

The kit for producing a keratoconjunctival cover sheet of this embodiment can include a component for imparting specific functions to the keratoconjunctival cover sheet that enables the keratoconjunctival cover sheet to be favorably used for the purpose of medical treatment, visual correction, a cosmetic application, or the like. For example, a container filled with an eye-drop solution containing a functional component such as a drug, a water-soluble component with a high refractive index, or a coloring agent may be provided, and the eye-drop solution of a water-soluble gelling agent may be contain such a functional component. Furthermore, a configuration in which a functional component such as a drug, a water-soluble component with a high refractive index, a coloring agent, or the like is dissolved or diluted just before use so as to be capable of being prepared just before use is also possible.

### Examples

Hereinafter, the present disclosure will be described in detail by use of examples. In these examples, keratoconjunctival cover sheets including sodium alginate as the water-soluble gelling agent and calcium ions as the metal ions are shown as example, but these examples are not limited thereto.

### Example 1. In-vitro production of keratoconjunctival cover sheet 1. Object

In this example, the production of a keratoconjunctival cover sheet was examined from the viewpoint of the gel strength and optical properties.

### 2. Materials

Materials of a keratoconjunctival cover sheet used in this example are summarized in Table 1.

**Table 1**

| | |
|---|---|
| Water-soluble gelling agent | Sodium alginate (viscosity range: 80 to 120 cp, 300 to 400 cp, 500 to 600 cp (10 g/L, 20°C), Wako Pure Chemical Industries, Ltd.) |
| Metal ions | Calcium chloride dihydrate (Wako Pure Chemical Industries, Ltd.) |
| Water-soluble component with high refractive index | Sucrose (Wako Pure Chemical Industries, Ltd.) |
| Solvent | Purified water |

### 3. Method

### 3-1. Preparation of aqueous solution of sodium alginate

To 100 mL of purified water, 1 g of sodium alginate powder was added, and was dissolved by stirring. At this time, sodium alginate powder with a viscosity within each range shown in section 2 above, and three types of aqueous solutions of sodium alginate (1 g/100 mL) that differed in viscosity were prepared.

### 3-2. Preparation of aqueous solution of sodium alginate and sucrose

An aqueous solution of sodium alginate and sucrose was prepared by mixing 20 g of the aqueous solution of sodium alginate (1 g/ 100 mL) with a viscosity within a range of 300 to 400 cp prepared in section 3-1, and 20 g of sucrose.

### 3-3. Preparation of aqueous solution of calcium chloride

To 100 mL of purified water, 7.4 g of calcium chloride dihydrate was added, and was dissolved by stirring. An aqueous solution of calcium chloride (7.4 g/100 mL) was thus prepared.

### 3-4. Gelation

Glass plates having a gently curved spherical surface that was a model of the surface of an eyeball were allowed to stand on paper on which letters had been printed, and 50 µL of each of three types of aqueous solutions of sodium alginate prepared in section 3-1 and the aqueous solution of sodium alginate and sucrose prepared in section 3-2 was pipetted and dripped onto the glass plate so as to have a circular shape. Subsequently, 50 µL of the aqueous solution of calcium chloride prepared in section 3-3 was pipetted and dripped onto each of the three types of aqueous solutions of sodium alginate and the aqueous solution of sodium alginate and sucrose on the glass plate.

### 4. Results

All of the three types of aqueous solutions of sodium alginate and the aqueous solution of sodium alginate and sucrose gelated soon after they were mixed with the aqueous solution of calcium chloride, and formed gel having a circular shape like a contact lens. The gel had a sufficiently high gel strength and could be pinched by tweezers (FIG. 1). The gel formed through gelation of the aqueous solution of sodium alginate and sucrose functioned as a lens and magnified the printed letters (FIG. 2).

It is known that ion-crosslinks are formed and cause gelation by alginic acid soon after calcium ions are added to an aqueous solution of sodium alginate, and it is thought that the same phenomenon also occurred in this example. Regarding the gel strength, the gel was not easily broken when pinched by tweezers, and it was thus confirmed that the gel was strong enough to be used as a keratoconjunctival cover sheet. The magnification of the printed letters in the system in which sucrose, which is a water-soluble component with a high refractive index, was added means that the refractive index increased due to the addition of sucrose, which shows that the refractive index of alginic acid gel can be controlled using sucrose.

### Example 2. In-vivo production of keratoconjunctival cover sheet

### 1. Object

In this example, the production of a keratoconjunctival cover sheet on the eye (cornea of keratoconjunctiva) of an experimental animal was examined.

### 2. Materials and experimental animal

The materials of a keratoconjunctival cover sheet used in this example were the same as those used in Example 1, and examination was performed using a rabbit as an experimental animal.

### 3. Method

### 3-1. Preparation of aqueous solution of sodium alginate, aqueous solution of sodium alginate and sucrose, and aqueous solution of calcium chloride

The three types of aqueous solutions of sodium alginate that differed in viscosity, the aqueous solution of sodium alginate and sucrose, and the aqueous solution of calcium chloride were prepared in the same manner as in sections 3-1 to 3-3 in Example 1.

### 3-2. Gelation on eye surface

50 µL of each of the three types of aqueous solutions of sodium alginate and the aqueous solution of sodium alginate and sucrose, which were prepared in section 3-1, were pipetted and dripped onto the eyeball of an anesthetized rabbit so as to have a circular shape. Subsequently, 50 µL of the aqueous solution of calcium chloride prepared in section 3-1 was pipetted and dripped onto each of the three types of aqueous solutions of sodium alginate and the aqueous solution of sodium alginate and sucrose on the eyeball.

### 4. Results

Similarly to Example 1, all of the three types of aqueous solutions of sodium alginate and the aqueous solution of sodium alginate and sucrose gelated on the eyeball soon after they were mixed with the aqueous solution of calcium chloride, and formed gel having a circular shape like a contact lens (inside the dotted line in FIG. 3). The gel had a sufficiently high gel strength and could be pinched by tweezers (a portion indicated by the arrow in FIG. 4).

It is known that ion-crosslinks are formed and cause gelation by alginic acid soon after calcium ions are added to an aqueous solution of sodium alginate, and it is thought that the same phenomenon also occurred on the eyeball in this example. Regarding the gel strength, the gel was not easily broken when pinched by tweezers, and it was thus confirmed that the gel was strong enough to be used as a keratoconjunctival cover sheet.

### Example 3. Adjustment of refractive index of keratoconjunctival cover sheet

### 1. Object

In this example, the adjustment of the refractive index of the keratoconjunctival cover sheet whose production had been examined in the previous example was examined.

### 2. Materials

Materials of a keratoconjunctival cover sheet used in this example are summarized in Table 2.

**Table 2**

| | |
|---|---|
| Water-soluble gelling agent | Sodium alginate (viscosity range: 300 to 400 cp (10 g/L, 20°C), Wako Pure Chemical Industries, Ltd., 190-09991) |
| Metal ions | Calcium chloride dihydrate (Wako Pure Chemical Industries, Ltd., 035-00455) |
| Water-soluble component with high refractive index | Sucrose (Wako Pure Chemical Industries, Ltd., 196-00015) |
| Solvent | Purified water |

### 3. Method

### 3-1. Preparation of aqueous solution of sodium alginate

To 100 mL of purified water, 1 g of sodium alginate powder was added, and was dissolved by stirring. An aqueous solution of sodium alginate (1 g/ 100 mL) was thus prepared.

### 3-2. Preparation of aqueous solution of calcium chloride

To 100 mL of purified water, 7.4 g of calcium chloride dihydrate was added, and was dissolved by stirring. An aqueous solution of calcium chloride (7.4 g/100 mL) was thus prepared.

### 3-3. Gelation and refractive index measurement

In order to control the refractive index, 100 mg, 200 mg, 400 mg, 600 mg, or 800 mg of sucrose, which serves as a water-soluble component with a high refractive index, was added to 1 mL of the aqueous solution of sodium alginate prepared in section 3-1 above and was dissolved by stirring. Subsequently, 100 µL of the obtained aqueous solution of sodium alginate and sucrose was dripped onto a measurement portion (maintained at 37°C) of a refractive index measurement apparatus (whose measurement principle is based on the principle of an Abbe's refractometer), and then 50 µL of the aqueous solution of calcium chloride prepared in section 3-2 above was dripped thereonto to cause gelation. The refractive index of the obtained gel was measured at 37°C.

### 4. Results

The results of the measurement of the refractive index were summarized in Table 3 and shown graphically in FIG. 5. FIG. 5 is a graph showing the change in refractive index depending on the sucrose concentration. The horizontal axis indicates the sucrose concentration (wt%), and the vertical axis indicates the refractive index. As shown in Table 3 and FIG. 5, it was revealed that the refractive index of the obtained gel changed in a sucrose concentration dependent manner. As confirmed in Example 1, it is shown from these results that the refractive index of the keratoconjunctival cover sheet can be controlled by adding the water-soluble component with a high refractive index, such as sucrose. Accordingly, it was revealed that the keratoconjunctival cover sheet can be used as a contact lens for visual correction, and contact lenses optimized for users can be provided by adjusting the concentration of the water-soluble component with a high refractive index, and the like to control the refractive index.

**Table 3**

| Sucrose concentration (wt%) | Refractive index |
|---|---|
| 0 mg/mL (0%) | 1.33212 |
| 100 mg/mL (9.9%) | 1.34566 |
| 200 mg/mL (17.58%) | 1.35751 |
| 400 mg/mL (29.16%) | 1.37690 |
| 600 mg/mL (38.40%) | 1.39384 |
| 800 mg/mL (44.98%) | 1.40665 |

### Example 4. Coloring of keratoconjunctival cover sheet

### 1. Object

In this example, the keratoconjunctival cover sheet whose production had been examined in the previous example was colored, and it was examined whether the colored keratoconjunctival cover sheet could be used for the purpose of a cosmetic application.

### 2. Materials

As the materials of a keratoconjunctival cover sheet used in this example, crocin (Tokyo Chemical Industries Co., Ltd., C1527) was used for coloring in addition to sodium alginate and calcium chloride hydrate used in Example 3. Crocin is a water-soluble carotenoid-based food dye that is contained in saffron and has been used as a food additive for a long time.

### 3. Method

### 3-1. Preparation of aqueous solution of sodium alginate and aqueous solution of calcium chloride

An aqueous solution of sodium alginate and an aqueous solution of calcium chloride were prepared in the same manner as in Example 3.

### 3-2. Gelation and coloring of gel

To 10 mL of the aqueous solution of sodium alginate prepared in section 3-1 above, 10 mg of crocin was added as a colorant for coloring, and was dissolved by stirring. A dark orange aqueous solution (crocin concentration: 0.1 wt%) was thus obtained. Subsequently, 50 µL of the obtained aqueous solution of sodium alginate and crocin was dripped onto a flat glass plate, and then 50 µL of the aqueous solution of calcium chloride prepared in section 3-1 above was dripped thereonto to form gel. Then, the external appearance of the gel was observed.

### 4. Results

The formation of a keratoconjunctival cover sheet with a yellow external appearance was confirmed (FIG. 6). It was revealed that the aqueous solution of the water-soluble gelling agent could be uniformly colored by mixing a colorant such as a food dye therewith, and the keratoconjunctival cover sheet could be used for the purpose of a cosmetic application such as a color contact lens.

### Example 5. Sustained drug release effect of keratoconjunctival cover sheet

### 1. Object

In this example, the sustained drug release effect of the keratoconjunctival cover sheet whose production had been examined in the previous example was examined, and it was examined whether the keratoconjunctival cover sheet could be used for the purpose of medical treatment.

### 2. Materials

As the materials of a keratoconjunctival cover sheet used in this example, levofloxacin (Tokyo Chemical Industries Co., Ltd., L0193) was used as a pharmaceutical component to form a sustained-release preparation, in addition to sodium alginate and calcium chloride hydrate used in Example 3. Levofloxacin is a new quinolone-based antibacterial drug used for treatment of conjunctivitis, prevention of postoperative infectious diseases, and the like.

### 3. Method

### 3-1. Preparation of aqueous solution of sodium alginate and aqueous solution of calcium chloride

An aqueous solution of sodium alginate and an aqueous solution of calcium chloride were prepared in the same manner as in Example 3.

### 3-2. Gelation and enclosure of drug

To 1 mL of the aqueous solution of sodium alginate prepared in section 3-1 above, 5 mg of levofloxacin was added as a pharmaceutical component to form a sustained-release preparation, and was dissolved by stirring. Subsequently, 50 µL of the obtained aqueous solution of sodium alginate and levofloxacin was dripped onto a flat glass plate, and then 50 µL of the aqueous solution of calcium chloride prepared in section 3-1 above was dripped thereonto to form gel. A keratoconjunctival cover sheet containing levofloxacin was thus produced.

### 3-3. Confirmation of sustained-release effect

The keratoconjunctival cover sheet produced in section 3-2 above was immersed in a beaker containing 100 mL of purified water. Aliquots were collected from the beaker at predetermined intervals (5, 10, 15, 20, and 30 minutes after the start of immersion), and the fluorescence spectra were measured using a fluorescence spectrum measurement apparatus to determine the fluorescence intensity of levofloxacin eluted from the keratoconjunctival cover sheet into the purified water. In this measurement, the excitation wavelength was set to 300 nm, and the maximum value in the fluorescence spectrum was obtained at a wavelength of 495 nm.

### 4. Results

FIGS. 7 and 8 show the results. FIG. 7 is a graph showing a temporal change in the fluorescence spectrum. The horizontal axis indicates the wavelength (nm) and the vertical axis indicates the fluorescence intensity. FIG. 8 is a graph showing a temporal change in the maximum fluorescence intensity (495 nm). The horizontal axis indicates the sustained-release time (minutes) and the vertical axis indicates the fluorescence intensity. As shown in FIGS. 7 and 8, it was confirmed that the keratoconjunctival cover sheet had a sustained drug release ability. It is known that a drug in a normal ocular instillation administration mode is subjected to immediate clearance due to the lacrimal fluid or blinking. In contrast, it was revealed that the keratoconjunctival cover sheet could be used as a sustained-release preparation of a drug for the purpose of medical treatment, and was expected to be used as a long-acting sustained-release preparation that can slowly release the drug for a longer period of time when the conditions were further examined.

### Example 6. In-vivo anti-allergic effect of keratoconjunctival cover sheet

### 1. Object

In this example, the anti-allergic effect of the keratoconjunctival cover sheet whose production had been examined in the previous example was examined in an animal testing system using a mouse, and it was examined whether the keratoconjunctival cover sheet could be used for the purpose of medical treatment.

### 2. Materials

Experimental animal: mouse (C57BL6/N)
Allergen: Ragweed Pollen (pollen of ragweed (PSI Polysciences, Inc., 07673-1): abbreviated as "RW" hereinafter) antigen
Adjuvant: Inject (registered trademark) Alum (Thermo SCIENTIFIC, 77161)
Sodium alginate and calcium chloride for producing keratoconjunctival cover sheet: sodium alginate and calcium chloride hydrate used in Example 3 were used.

### 3. Method

### 3-1. Production of allergic conjunctivitis mouse model

After the abdominal region of a mouse (C57BL6/N) was sterilized using 70% ethanol, a triple anesthetic combination (0.3 mg/kg of medetomidine (domitor (registered trademark)) + 4 mg/kg of midazolam + 5 mg/kg of butorphanol (vetorphale (registered trademark))) was intraperitoneally administered so as to allow the mouse not to feel pain. Then, a suspension (50 µL) of RW (1.5 mg) and Inject (registered trademark) Alum (1.5 ml) was subcutaneously injected to the posterior footpad and the root of tail of the mouse to sensitize the mouse to the RW antigen. An RW antigen-specific allergic conjunctivitis mouse model was thus produced.

### 3-2. Production of keratoconjunctival cover sheet and antigen challenge

Antigen challenge was carried out from the tenth to the fourteenth day after the antigen sensitization described in section 3-1 above. Specifically, test group (B) (n=4) was prepared in which a keratoconjunctival cover sheet was produced and provided on the eye (cornea or keratoconjunctiva) of the RW-specific allergic conjunctivitis mouse model produced in section 3-1 above. The keratoconjunctival cover sheet was produced through ocular instillation using an aqueous solution of sodium alginate and an aqueous solution of calcium chloride prepared in the same manner as in Example 3, in accordance with the method described in Example 2 above. Moreover, group (A) (n=4) that was not provided with the keratoconjunctival cover sheet, and control group (C) (n=2) that was not subjected to the ocular instillation for antigen challenge were prepared. Subsequently, the antigen challenge was carried out by applying the RW antigen to the eyes of the mice of the groups ((A) and (B)) using a solution of 2 mg RW/10 mL PBS. In all cases, a dose of ocular instillation was 10 µL/eye.

### 3-3. Histomorphological evaluation

The mice of the groups were euthanized by administration of an excessive amount of anesthesia 24 hours after the antigen challenge described in section 3-2 above, and the eyeballs with accompanying structures of eyelids and eyeballs were extracted, immersed in 10% formalin neutral buffer solution (Wako Pure Chemical Industries, Ltd., 062-01661) for a day and a night, and embedded in Tissue-Tek O.C.T. Compound (Sakura Finetek Japan Co., Ltd.). Then, sections with a thickness of 10 µm were formed using a cryostat. Subsequently, the presence and the number of granulocytes were histomorphologically evaluated using a predetermined Giemsa staining method (immersed in 20-fold dilution of GS500 (Sigma-Aldrich) for 30 minutes).

### 4. Results

FIG. 9 shows the results. FIG. 9 shows micrographs of Giemsa-stained conjunctival portions. Panel A shows the result from group (A) that was not provided with the keratoconjunctival cover sheet. Panel B shows the result from test group (B) that was provided with the keratoconjunctival cover sheet. Panel C shows the result from control group (C) that was not subjected to antigen challenge. Regarding group (A) that was not provided with the keratoconjunctival cover sheet, a large number of groups of granulocytes (gray granules in the diagram) that had been stained in a purplish color were observed in the conjunctival portion (panel A in FIG. 9). On the other hand, regarding group (B) that was provided with the keratoconjunctival cover sheet and control group (C) that was not subjected to the antigen challenge, a small number of groups of granulocytes (gray granules in the diagram) that had been stained a purplish color were observed in the conjunctival portion (panels B and C in FIG. 9). It is thought that this is because the keratoconjunctival cover sheet physically covered the eye surface and physically blocked the antigen challenge. Accordingly, it was shown that the keratoconjunctival cover sheet had an effect of enabling the prevention of onset of allergy.

### Example 7. Water-retention effect of keratoconjunctival cover sheet

### 1. Object

In this example, the water-retention effect of the keratoconjunctival cover sheet whose production had been examined in the previous example was examined.

### 2. Materials

As the materials of a keratoconjunctival cover sheet used in this example, sodium hyaluronate (Wako Pure Chemical Industries, Ltd., Cat. No 089-10343), which is a moisturizing component, and silicone oil (Shin-Etsu Chemical Co., Ltd., KF-96-50CS) for forming a surface coating was used to improve the water retention of the keratoconjunctival cover sheet, in addition to sodium alginate and calcium chloride hydrate used in Example 3.

### 3. Method

### 3-1. Preparation of aqueous solution of sodium alginate

In 50 mL of purified water, 500 mg of sodium alginate was dissolved. An aqueous solution of sodium alginate was thus produced (solution 1).

### 3-2. Preparation of aqueous solution of calcium chloride

In 20 mL of purified water, 1480 mg of calcium chloride was dissolved. An aqueous solution of calcium chloride was thus produced (solution 2).

### 3-3. Gelation and confirmation of water retention

Keratoconjunctival cover sheets of (1) to (3) below were produced, and the water retention ratio was confirmed by comparing the control (1).
(1) Keratoconjunctival cover sheet (control): After 50 µL of the aqueous solution of sodium alginate prepared in section 3-1 above was dripped onto slide glass, 50 µL of the aqueous solution of calcium chloride prepared in section 3-2 above was dripped thereonto to form gel, and a keratoconjunctival cover sheet was thus produced.
(2) Silicone oil-containing keratoconjunctival cover sheet: To 50 µL of the aqueous solution of sodium alginate prepared in section 3-1 above, 50 µL of an aqueous solution dissolving 50 µL of silicone oil was added, and 100 µL of the obtained aqueous solution of sodium alginate and silicone oil was collected and dripped on slide glass. Thereafter, 50 µL of the aqueous solution of calcium chloride prepared in section 3-2 above was dripped thereonto to form gel, and a silicone oil-containing keratoconjunctival cover sheet was thus produced.
(3) Hyaluronic acid-containing keratoconjunctival cover sheet: To 50 µL of the aqueous solution of sodium alginate prepared in section 3-1 above, 50 µL of an aqueous solution dissolving 1 mg/50 µL hyaluronic acid was added, and 100 µL of the obtained aqueous solution of sodium alginate and hyaluronic acid was collected and dripped on slide glass. Thereafter, 50 µL of the aqueous solution of calcium chloride prepared in section 3-2 above was dripped thereonto to form gel, and a hyaluronic acid-containing keratoconjunctival cover sheet was thus produced.

Regarding the water retention ratio of the keratoconjunctival cover sheet, after the weights of the keratoconjunctival cover sheets (1) to (3) above were measured in advance, the keratoconjunctival cover sheets were allowed to stand at room temperature, the temporal changes in weight were measured, and the changes in weight were evaluated.

### 4. Results

FIG. 10 shows the results. FIG. 10 is a graph showing temporal changes in the water content ratio of the keratoconjunctival cover sheets. The horizontal axis indicates the elapsed time (minutes) and the vertical axis indicates the water content ratio (%). It was confirmed from the results of the keratoconjunctival cover sheet (control) (1) that water evaporated and the weight decreased as time elapsed. When the keratoconjunctival cover sheets (2) and (3) are compared, the temporal change in weight was the smallest in the case of the keratoconjunctival cover sheet on which a coating made of the oil contained in the silicone oil-containing keratoconjunctival cover sheet (2) was formed. That is, this means that, in the silicone oil-containing keratoconjunctival cover sheet (2), the water evaporation from the keratoconjunctival cover sheet was effectively suppressed due to the oil coating effect, and it was revealed that using silicon oil as the coating of the keratoconjunctival cover sheet was effective. Furthermore, the water evaporation from the hyaluronic acid-containing keratoconjunctival cover sheet (3) was suppressed compared with the keratoconjunctival sheet (1), and therefore, it was revealed that adding a moisturizing component such as hyaluronic acid to a keratoconjunctival cover sheet exhibits an effect of suppressing water evaporation from the keratoconjunctival cover sheet.

### Example 8. Sustained drug release effect of keratoconjunctival cover sheet

### 1. Object

In this example, in addition to Example 5, the sustained drug release effect of the keratoconjunctival cover sheet whose production had been examined in the previous example was examined, and it was examined whether the keratoconjunctival cover sheet could be used for the purpose of medical treatment. Here, the sustained drug release effect was examined using a therapeutic drug for dry eye, rebamipide.

### 2. Materials

As the materials of a keratoconjunctival cover sheet used in this example, a therapeutic drug for dry eye, rebamipide (Tokyo Chemical Industries Co., Ltd., R0085) was used as a pharmaceutical component to form a sustained-release preparation, in addition to sodium alginate and calcium chloride hydrate used in Example 3.

### 3. Method

### 3-1. Preparation of aqueous solution of sodium alginate and aqueous solution of calcium chloride

An aqueous solution of sodium alginate and an aqueous solution of calcium chloride were prepared in the same manner as in Example 7.

### 3-2. Formation of nanoparticles of drug, rebamipide

Rebamipide is a drug that is poorly soluble in water. Therefore, nanoparticles of the drug were formed in order to disperse the drug in water. To 50 ml of purified water, 50 mg of the drug was mixed, and ultrasonic waves were applied to the mixture for 30 minutes using a sonicator. A suspension was thus produced. Subsequently, the suspension was passed through a syringe filter with a pore diameter of 0.8 µm, and an aqueous suspension of nanoparticles of the drug was thus obtained. The production of nanoparticles was confirmed using a scanning electron micrograph (FIG. 11).

### 3-3. Gelation and enclosure of drug

To 10 ml of the aqueous suspension of the rebamipide nanoparticles prepared as a pharmaceutical component to form a sustained-release preparation in section 3-2 above, 100 mg of sodium alginate was added, and was dissolved by stirring. Subsequently, 100 µL of the obtained aqueous solution of sodium alginate and the drug was dripped onto the bottom of a quartz cell for fluorescence intensity measurement, and then 100 µL of the aqueous solution of calcium chloride prepared in section 3-1 above was dripped thereonto to form gel. A keratoconjunctival cover sheet containing the drug was thus produced on the bottom of the quartz cell.

### 3-4. Confirmation of sustained-release effect

To the quartz cell containing the keratoconjunctival cover sheet produced in section 3-3 above, 2 mL of purified water was poured, and thereby the keratoconjunctival cover sheet was immersed in the purified water and allowed to stand. The fluorescence spectra were measured using a fluorescence spectrum measurement apparatus at predetermined intervals (10, 20, 30, 40, and 60 minutes after the start of immersion) to determine the fluorescence intensity of the drug eluted from the keratoconjunctival cover sheet into the purified water. Sustained drug release from the keratoconjunctival cover sheet was evaluated by performing the measurement over time. In this measurement, the excitation wavelength was set to 270 nm, and the maximum value in the fluorescence spectrum was obtained at a wavelength of 370 nm.

### 4. Results

FIGS. 12 and 13 show the results. FIG. 12 is a graph showing a temporal change in the fluorescence spectrum. The horizontal axis indicates the wavelength (nm) and the vertical axis indicates the fluorescence intensity. As shown in FIG. 12, the fluorescence intensity of the fluorescence spectrum increased as time elapsed. FIG. 13 is a graph showing a temporal change in the maximum fluorescence intensity. The horizontal axis indicates the sustained-release time (minutes) and the vertical axis indicates the fluorescence intensity. In FIG. 13, the maximum values of fluorescence intensity (wavelength: 370 nm) in FIG. 12 are plotted. The results shown in FIGS. 12 and 13 mean that the drug contained in the keratoconjunctival cover sheet was slowly released into the purified water, and it was confirmed that the keratoconjunctival cover sheet had a sustained drug release ability. It was also confirmed from these results together with the results of Example 5 that the sustained drug release ability could be exhibited irrespective of the type and form of a drug. Accordingly, it was revealed that the keratoconjunctival cover sheet could be used as a sustained-release preparation of a drug for the purpose of medical treatment.

### Industrial Applicability

The present disclosure relates to a keratoconjunctival cover sheet and a method for producing a keratoconjunctival cover sheet, and can be favorably used in the fields of medical treatment, visual correction, and a cosmetic application, and also favorably used in sheets for sustained drug release, contact lenses for visual correction, color contact lenses, and the like.

## Claims

1. A keratoconjunctival cover sheet constituted by a gel layer that includes metal ions and a water-soluble gelling agent exhibiting a gelation ability in the presence of the metal ions, and has a surface area capable of covering a cornea or keratoconjunctiva.

2. The keratoconjunctival cover sheet according to claim 1, wherein the water-soluble gelling agent is alginic acid or an alginic acid derivative, and the metal ions are positive ions or negative ions.

3. The keratoconjunctival cover sheet according to claim 2, wherein the metal ions are calcium ions.

4. The keratoconjunctival cover sheet according to any one of claims 1 to 3, which is used for protection from an external environment.

5. The keratoconjunctival cover sheet according to any one of claims 1 to 4, which contains a therapeutically effective amount of a drug in the gel layer and is used to slowly release the drug.

6. The keratoconjunctival cover sheet according to any one of claims 1 to 5, wherein the gel layer is constituted by a contact lens with a predetermined refractive index.

7. The keratoconjunctival cover sheet according to claim 6, which contains a water-soluble component with a high refractive index in the gel layer and is used for visual correction.

8. The keratoconjunctival cover sheet according to claim 7, wherein the water-soluble component with a high refractive index is selected from sugars, polyhydric alcohols, water-soluble polymers, and mixtures thereof.

9. The keratoconjunctival cover sheet according to any one of claims 1 to 8, wherein the gel layer contains an oil component or a moisturizing component.

10. A method for producing a keratoconjunctival cover sheet, comprising:
a step of applying a water-soluble gelling agent to an eye, in which a water-soluble gelling agent exhibiting a gelation ability in the presence of metal ions is applied to an anterior ocular segment; and
a step of applying metal ions to an eye, in which the metal ions are applied to the anterior ocular segment,
wherein gelation by the water-soluble gelling agent is caused by the metal ions, and a gel layer is formed on a cornea or keratoconjunctiva.

11. The method for producing a keratoconjunctival cover sheet according to claim 10, wherein the step of applying a water-soluble gelling agent to an eye includes a step of applying a therapeutically effective amount of a drug to the anterior ocular segment.

12. The method for producing a keratoconjunctival cover sheet according to claim 10 or 11, wherein the step of applying a water-soluble gelling agent to an eye includes a step of applying a water-soluble component with a high refractive index to the anterior ocular segment.

13. The method for producing a keratoconjunctival cover sheet according to claim 12, wherein gel layers including a single component or gel layers including a plurality of components are laminated by repeating the step of applying a water-soluble gelling agent to an eye and the step of applying metal ions to an eye to control a refractive index.

14. The method for producing a keratoconjunctival cover sheet according to claim 12 or 13, wherein at least one of factors of the gel layer associated with a refractive index, such as viscosity, magnetic permeability, dielectric constant, electric conductivity, and gel thickness, is adjusted to control a refractive index.

15. The method for producing a keratoconjunctival cover sheet according to any one of claims 10 to 14, further comprising a step of applying an oil component or a moisturizing component to the anterior ocular segment.
